# EUROPEAN PATENT APPLICATION

(11) **EP 4 840 954 A1**
(43) Date of publication of application: **18.11.2026**
(21) Application number: 26174303.3
(22) Date of filing: 23.04.2026
(51) Int. Cl.: C12N 15/10

(54) **METHOD FOR LIGATING AN ADAPTOR TO AN RNA FRAGMENT ASSOCIATED WITH A RIBOSOME AND KIT THEREOF**

(30) Priority: 16.05.2025 IT 202500011128
(71) Applicant: Immagina Biotechnology S.r.l., 38057 Pergine Valsugana (TN) (IT); Consiglio Nazionale Delle Ricerche - CNR, 00185 Roma (IT); Università Degli Studi Di Trento, 38122 Trento (IT)
(72) Inventor: VIERO, Gabriella, Roma (RM) (IT); BRUNO, Ilaria, Trento (TN) (IT); PERRUCCI, Cecilia, Roma (RM) (IT); TOME', Gabriele, Roma (RM) (IT); SUSIN, Giorgia, Roma (RM) (IT); PERENTHALER, Elena, Roma (RM) (IT); LAURIA, Fabio, Roma (RM) (IT)
(74) Representative: Buzzi, Notaro & Antonielli d'Oulx S.p.A.

(57) **Abstract**

Method for ligating an adaptor to a ribosome-embedded RNA Fragment (REF) from a cell population and kit thereof, wherein the method comprises the following steps:
(i) performing a lysis of the cell population, obtaining a cell lysate;
(ii) digesting the cell lysate with an endonuclease, obtaining a digested lysate;
(iii) inhibiting the endonuclease in the digested lysate, obtaining an inhibited lysate;
(iv) incubating the inhibited lysate with an adaptor in conditions to allow ligation of the adaptor to the REF, and obtaining an adaptor-REF complex, wherein the adaptor has formula (I):

5' OH-Nₓ-C1-L1-A_{z}-PR1-N_{y}-C2-B-OH 3' (I).

## Description

### Field of the invention

The present description concerns a novel method for ligating an adaptor to an RNA fragment embedded within a ribosome and kit thereof.

### Background

Several experimental approaches have been developed to investigate the complete set of mRNAs engaged in translation across both cell lines and tissues. Among these, the most used assay that generates genome-wide descriptions of protein synthesis efficiency is ribosome profiling (RiboSeq) (Ingolia et al., 2009). By providing a positional landscape of ribosomes on RNA at single-nucleotide resolution, this approach has become instrumental in uncovering previously uncharacterized layers of gene expression regulation. These insights have broadened the understanding of translated RNAs, including conventional and unconventional open reading frames (ORFs), such as upstream and downstream ORFs, as well as the overall peptide-coding potential of the transcriptome.

RiboSeq involves the generation of 30-nucleotide-long ribosome-protected RNA fragments (RPFs), followed by their extraction and, often, gel-based size selection. These RPFs are then converted into a doublestranded DNA library suitable for sequencing. The resulting data are highly informative and serve numerous applications, including mapping ribosome positions along transcripts at single-nucleotide resolution, quantifying ribosome engagement on mRNAs, identifying RNAs with canonical or non-canonical coding potential, and analyzing ribosome-based regulatory mechanisms in translation. Additionally, when combined with precipitation techniques, RiboSeq enables the study of translational control by ribosome-associated proteins, cell type-specific ribosomal populations, translation fidelity, gene-specific elongation rates, and the active pool of mRNAs undergoing translation.

Despite its widespread application, RiboSeq presents several challenges, often resulting in suboptimal data quality (Wang and Mao, 2023). The technique remains complex and labor-intensive, typically requiring substantial amounts of starting material to generate sufficient RPFs for successful library preparation. Over time, refinements have been made to various protocol steps, including cell lysis, RNase digestion, ribosome isolation, and library construction. Circularization-based methods, which involve RNA ligation and DNA or RNA circularization, have faced inefficiencies and inaccuracies at the 5' end (Ingolia et al., 2009; McGlincy & Ingolia, 2017). Dual-ligation methods have been introduced to mitigate these issues by ligating adaptors to both 3' and 5' ends of RNA fragments, though challenges such as ligation biases and adaptor self-ligation still affect efficiency (VanInsberghe et al., 2021). More recently, template switching-based methods have gained traction due to their ability to process low-input samples, but they introduce variability and sequence biases that impact the accuracy of ribosome occupancy profiling.

While both dual-ligation and template-switching approaches hold promise for low-input and single-cell applications, addressing inherent biases and optimizing each step remain critical for producing reliable, high-quality datasets. As a result, RiboSeq is still far from being a user-friendly technique, particularly for applications requiring minimal input material. Its complexity and the need for extensive optimization at multiple stages pose significant barriers to its widespread adoption in high-throughput research and clinical settings.

### Object and summary of the invention

The object of the present invention is to provide a novel and highly efficient solution for beyondresolution ribosome profiling, overcoming the limitations of traditional RiboSeq techniques, and enabling unprecedented accuracy and sensitivity in translational studies.

According to the invention, the above object is achieved thanks to the method specified in the ensuing claims, which are understood as forming an integral part of the present description.

In one embodiment, the present invention concerns a method for ligating an adaptor to a ribosome-embedded RNA Fragment (REF) as defined in claim 1.

In one embodiment, the present invention concerns a kit for performing the method for ligating an adaptor to a REF as defined in claim 10.

The method herein disclosed (named RiboWich) enables high efficiency of adaptor ligation to REFs and produces high-quality sequencing libraries. This streamlined workflow reduces background noise, enhances data quality, and facilitates the discovery of unconventional ORFs and cell type-specific alternative translational events.

The RiboWich method allows for:
- Enhanced Ribosome Profiling: achieving superior translational resolution and identifying conventional and unconventional ORFs.
- High-Throughput applications: facilitating scalable and cost-effective multiplexed ribosome profiling experiments directly in cellular or tissue lysates.

By streamlining ribosome profiling and significantly elevating data quality, the RiboWich method establishes a new benchmark for advanced research in cell and molecular biology and opens pathways for innovative applications in translational medicine.

### Brief description of the drawings

The invention will now be described, by way of example only, with reference to the enclosed figures of the drawing, wherein:

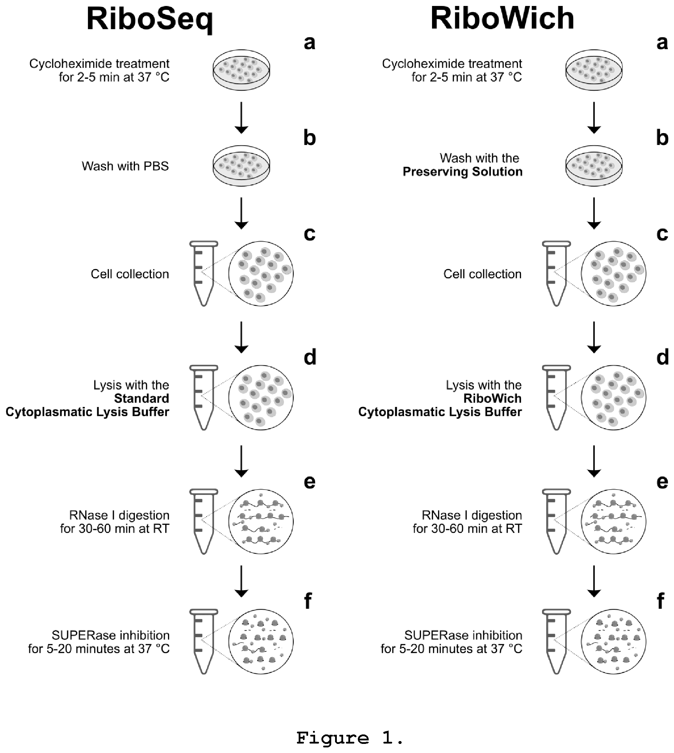

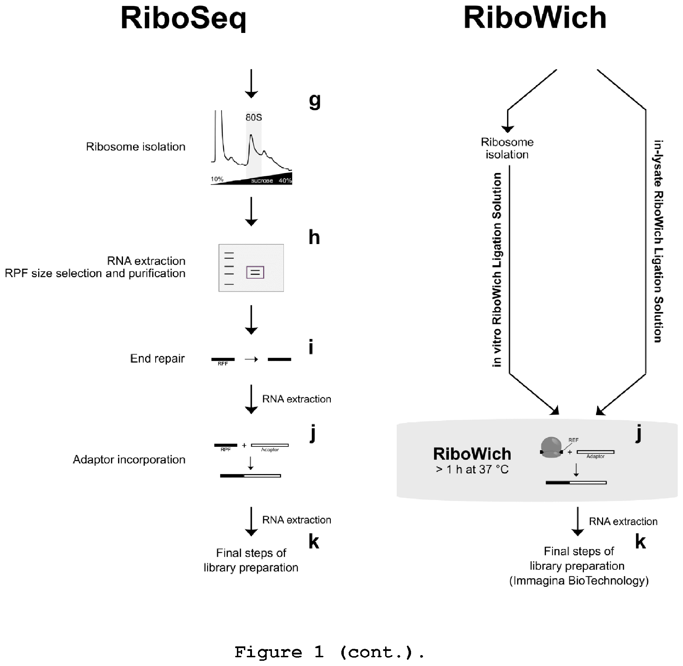

- **Figure 1****. Detailed comparison of RiboSeq and RiboWich protocols.** Detailed illustration of the RiboSeq (left) and RiboWich (right) workflows, highlighting the shared steps and the key differences between the two methods.

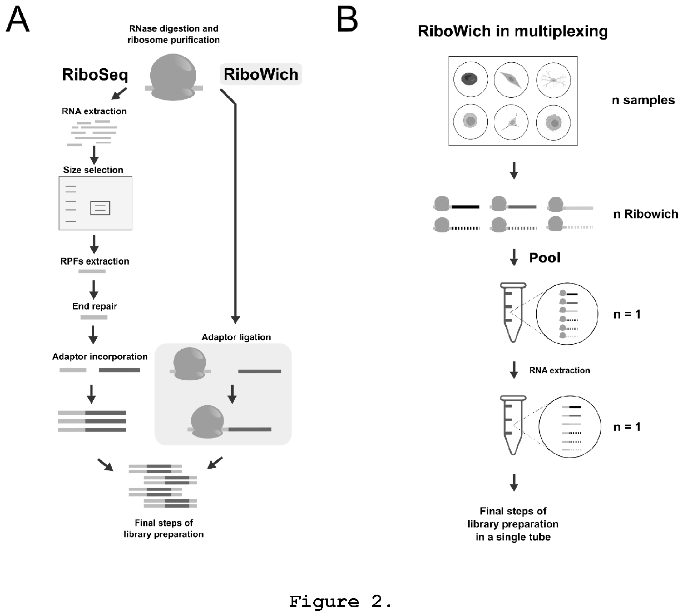

- **Figure 2****. Schematic comparison of RiboSeq and RiboWich methods. (A)** As a common step to initiate ribosome profiling, cytoplasmatic lysates are treated with RNase, and ribosomes are purified. Existing RiboSeq protocols typically include RNA extraction, polyacrylamide gel size selection, and extraction of RPFs. From the purified and nude RPFs, the steps of library preparation begin. Distinctively, RiboWich ligates the adaptor directly to the REFs. Upon ligation, RNA is extracted, and the following steps of library preparation are carried out. **(B)** Scheme of multiplexingbased RiboWich experimental design. Different samples are tagged with different barcoded adaptors. Upon the ligation of barcoded adaptors to REFs, samples can be pooled together, and the following steps of library preparation are carried out in one single tube.

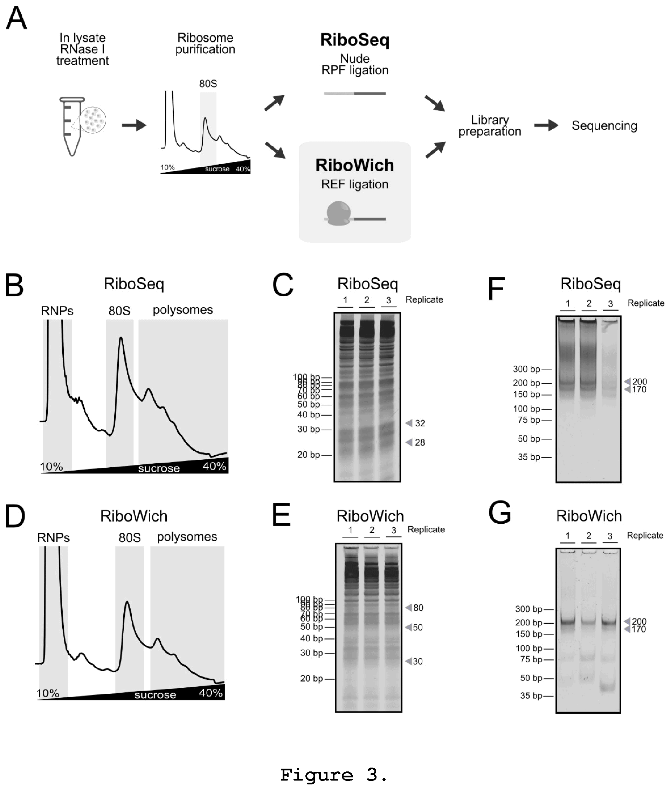

- **Figure 3****. Comparison of RiboSeq and RiboWich. (A)** Experimental workflow for in parallel RiboSeq and RiboWich. **(B)** Representative polysome profiles from HEK293T cellular lysates treated with RNase I and used for RiboSeq library preparation. **(C)** Gel electrophoresis for size selection and purification of the RPFs (30nt) obtained from three replicates, used for RiboSeq library preparation. **(D)** Representative polysome profiles from HEK293T cellular lysates treated with RNase I and used for RiboWich library preparation. **(E)** Gel electrophoresis for size selection and purification of the RiboWich ligation product (80nt) obtained from three replicates, used for RiboWich library preparation. **(F,G)** Gel electrophoresis for size selection and purification of the final libraries (200bp) obtained with RiboSeq (F) and RiboWich (G), for the three replicates. The bands at 170bp represent byproducts coming from the different reactions of library preparation.

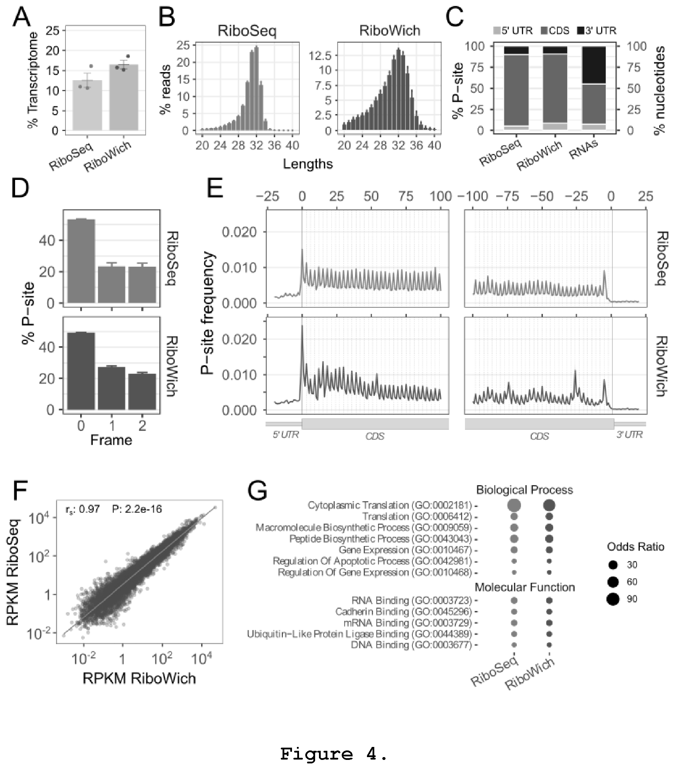

- **Figure 4****. Comparison between RiboSeq and RiboWich performed in HEK293T. (A)** Percentages of reads aligning to the transcriptome for RiboSeq and RiboWich. **(B)** Percentage distribution of read lengths for RiboSeq (left) and RiboWich (right). **(C)** Percentages of P-sites mapping on the 5' UTR, CDS, and 3' UTR for RiboSeq (left) and RiboWich (middle). Percentages of region lengths in mRNA sequences are reported as reference (right). **(D)** Percentages of P-sites mapping to the three reading frames (0, 1, 2) for RiboSeq (top) and RiboWich (bottom). **(E)** Meta-profiles showing the frequency of P-sites mapping at the beginning and the end of the coding sequence in RiboSeq (top) and RiboWich (bottom). **(F)** Correlations of RPKM per protein-coding transcripts (*n = 20 499*) between RiboSeq and RiboWich. The Spearman correlation and the statistical significance based on the two-tailed William's test are reported. **(G)** Enrichment analysis based on Gene Ontology Biological Process (top) and Molecular Function (bottom) terms of the top covered 5% genes. All terms display a statistically significant enrichment (adjusted p-value < 0.05), and the dots' size is proportional to the Odds Ratio. In all panels except (G), results are shown as the average among n *=* 3 biologically independent samples, and the Standard Error of the Mean (SEM) is reported in (A), (B), (D), (E).

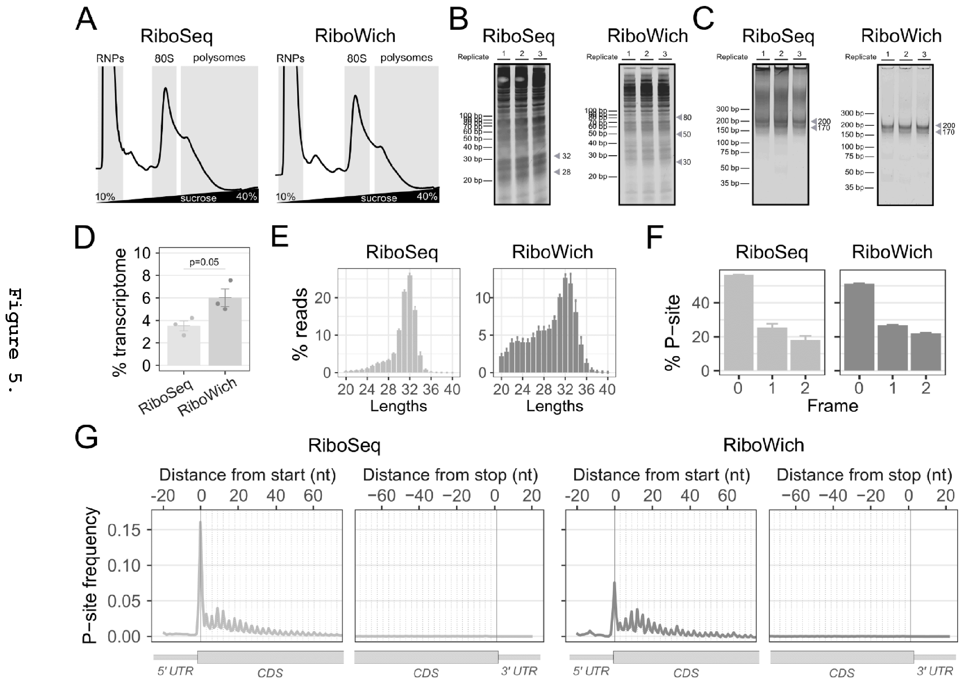

- **Figure 5****. Comparison between RiboSeq and RiboWich performed in HEK293T treated with harringtonine. (A)** Representative polysome profiles from HEK293T cells treated with harringtonine, lysed, and incubated with RNase I, used for RiboSeq (left) and RiboWich (right) library preparation. **(B)** Gel electrophoresis for size selection and purification of RPFs (28-32nt) used for traditional RiboSeq (left) and for size selection and purification of the RiboWich ligation products (80nt) used for the RiboWich (right), in the three replicates treated with harringtonine. **(C)** Gel electrophoresis for size selection and purification of the final library (200bp) obtained with the RiboSeq (left) and RiboWich (right) protocols, for the three replicates treated with harringtonine. The bands at 170bp represent byproducts coming from the different reactions of library preparation. **(D)** Percentages of reads aligning to the transcriptome for RiboSeq and RiboWich. The statistical difference between RiboSeq and RiboWich was tested using two-sided T-tests (P-value is reported). **(E)** Percentage distribution of read lengths in RiboSeq (left) and RiboWich (right) datasets. **(F)** Percentages of P-sites mapping to the three reading frames (0, 1, 2) for RiboSeq (left) and RiboWich (right). **(G)** Meta-profiles showing the frequency of P-sites mapping at the beginning and the end of the coding sequence in RiboSeq (left) and RiboWich (right). In (D), (E), (F), and (G), results are shown as the average ± Standard Error of the Mean (SEM) among *n = 3* biologically independent samples.

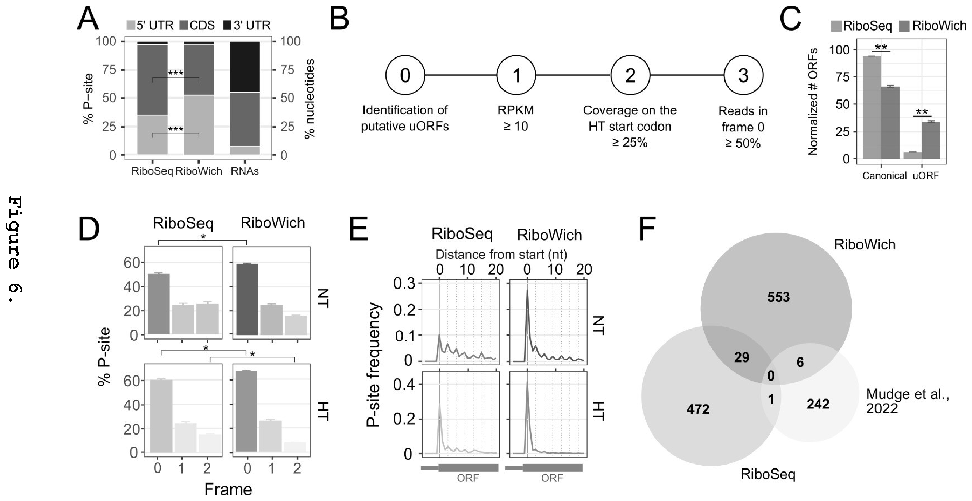

- **Figure 6****. Upstream ORFs in RiboSeq and RiboWich. (A)** Percentages of P-sites mapping on the 5' UTR, CDS, and 3' UTR for RiboSeq (left) and RiboWich (middle) in HEK293T cells treated with harringtonine. Percentages of region lengths in mRNA sequences are represented as reference (right). **(B)** Schematic representation of the workflow used to define active uORFs. **(C)** Number of active uORFs normalized against the number of canonical ORFs. **(D)** Percentages of P-sites mapping to the three reading frames (0, 1, 2) for RiboSeq (left) and RiboWich (right), NT (top) and HT (bottom) samples. **(E)** Meta-profiles showing the frequency of P-sites mapping at the beginning of the coding sequence for RiboSeq (left) and RiboWich (right), NT (top) and HT (bottom) samples. **(F)** Intersection of the active uORFs identified in RiboSeq and RiboWich and the uORFs associated with at least one peptide detected in proteomics studies according to Mudge et al., 2022. In (A), (C), (D), and (E), results are shown as the average among *n = 3* biologically independent samples, and the Standard Error of the Mean (SEM) is reported, except for (A). All statistical differences between RiboSeq and RiboWich were tested using two-sided T-tests (P-value: * < 0.05, ** < 0.01, *** < 0.001).

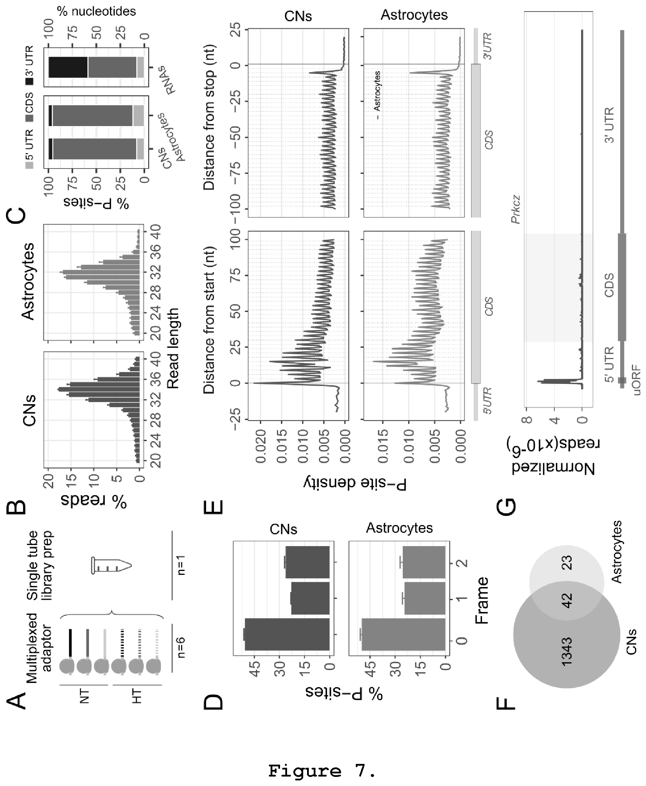

- **Figure 7****. RiboWich data from low-input NT primary CNs and astrocytes. (A)** Schematic representation of the RiboWich multiplexing workflow. **(B)** Percentage distribution of read lengths for CNs (left) and astrocytes (right). **(C)** Percentages of P-sites mapping on the 5' UTR, CDS, and 3' UTR for CNs (left) and astrocytes (middle). Percentages of region lengths in mRNA sequences are reported as reference (right). **(D)** Percentages of P-sites mapping to the three reading frames (0, 1, 2) for CNs (top) and astrocytes (bottom). **(E)** Meta-profiles showing the frequency of P-sites mapping at the beginning and the end of the coding sequence in CNs (top) and astrocytes (bottom). **(F)** Intersection of active uORFs detected in primary CNs and astrocytes. **(G)** Read coverage profiles for the CNs-specific Prkcz transcript, normalized against the library size for each replicate. In (B), (C), (D), (E), and (G), results are shown as the average among n = 2 (CNs) or n = 3 (astrocytes) biologically independent samples, and the Standard Error of the Mean (SEM) is reported, except for (C).

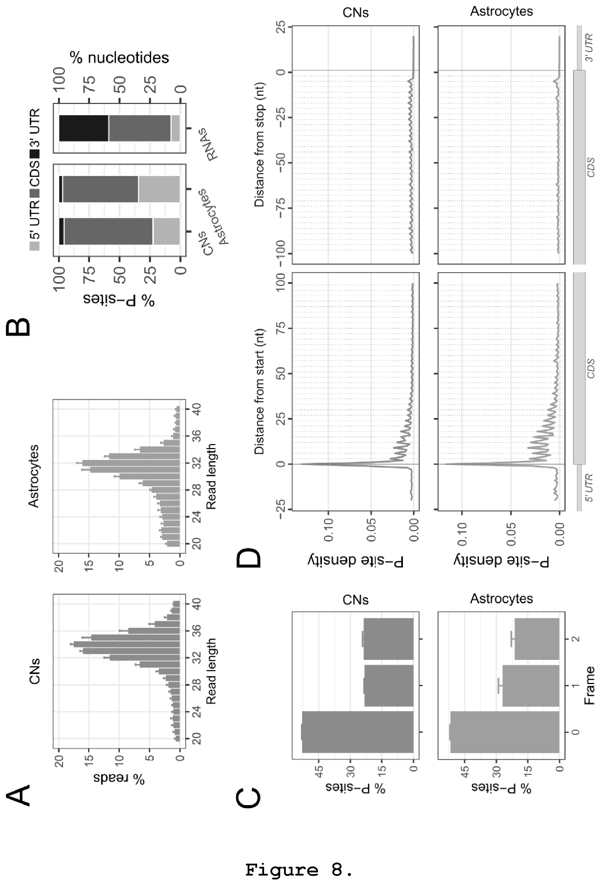

- **Figure 8****. RiboWich data from low-input primary CNs and astrocytes samples treated with harringtonine. (A)** Percentage distribution of read lengths for CNs (left) and astrocytes (right). **(B)** Percentages of P-sites mapping on the 5' UTR, CDS, and 3' UTR for CNs (left) and astrocytes (middle). Percentages of region lengths in mRNA sequences are reported as reference (right). **(C)** Percentages of P-sites mapping to the three reading frames (0, 1, 2) for CNs (top) and astrocytes (bottom). **(D)** Meta-profiles showing the frequency of P-sites mapping at the beginning and the end of the coding sequence in CNs (top) and astrocytes (bottom). In all panels, results are shown as the average among n = 2 (CNs) or *n* = 3 (astrocytes) biologically independent samples, and the Standard Error of the Mean (SEM) is reported, except for (B).

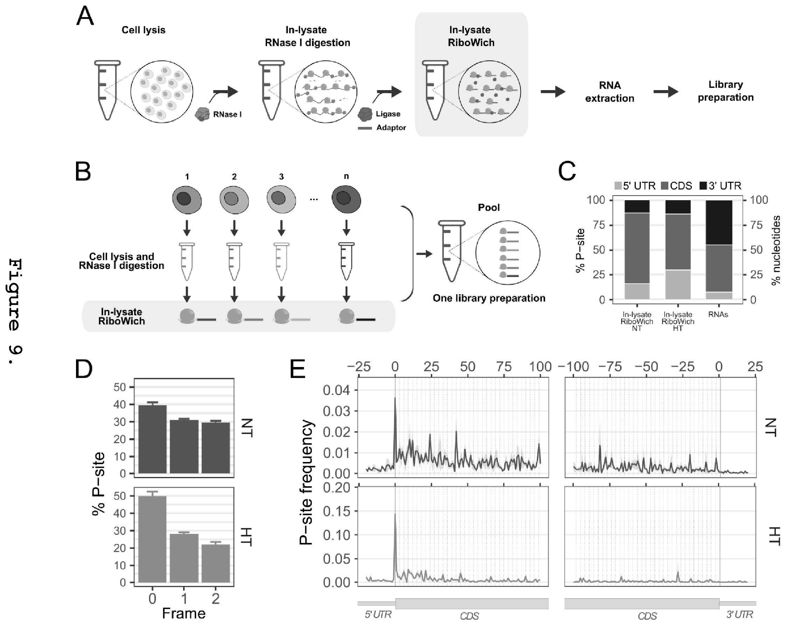

- **Figure 9****. In-lysate RiboWich. (A)** Schematic overview of the in-lysate RiboWich workflow. Cells are lysed, and the lysates are treated with RNase I. Next, the ligation to REFs occurs directly in the lysate. After ligation, RNA is extracted, and the following library preparation steps are carried out. **(B)** Schematic overview of the multiplexed in-lysate RiboWich. During ligation, different samples are tagged using different barcoded adaptors. Upon barcoding, samples can be pooled together, and the following library preparation steps are carried out in one single tube. **(C)** Percentages of P-sites mapping on the 5' UTR, CDS, and 3' UTR for in-lysate RiboWich NT (left) and in-lysate RiboWich HT (middle). Percentages of region lengths in mRNA sequences are reported as reference (right). **(D)** Percentages of P-sites mapping to the three reading frames (0, 1, 2) for in-lysate RiboWich NT (top) and in-lysate RiboWich HT (bottom). **(E)** Meta-profiles showing the frequency of P-sites mapping at the beginning and the end of the coding sequence in in-lysate RiboWich NT (top) and in-lysate RiboWich HT (bottom). In (C), (D), and (E), results are shown as the average among n = *3* biologically independent samples, and the Standard Error of the Mean (SEM) is reported in (D) and (E).

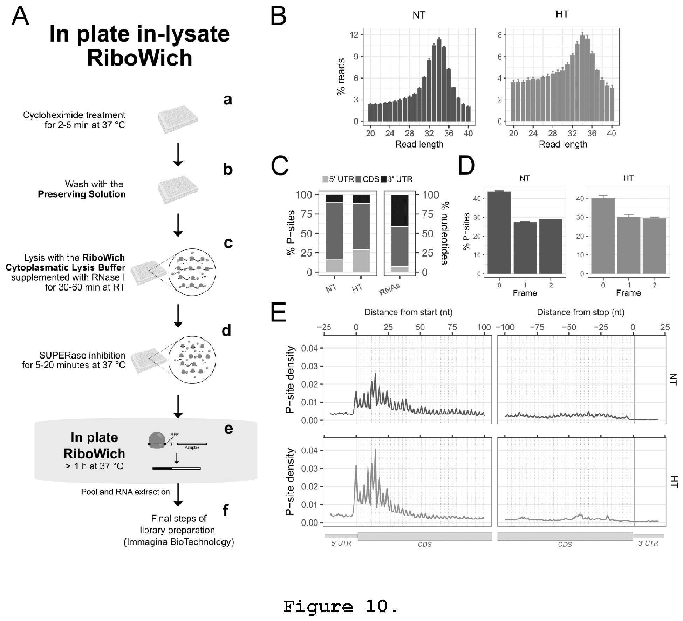

- **Figure 10****. RiboWich data from very low-input HEK293T NT and HT. (A)** Detailed protocol of in plate in-lysate RiboWich. **(B)** Percentage distribution of read lengths in NT (left) and HT (right) samples. **(C)** Percentages of P-sites mapping on the 5' UTR, CDS, and 3' UTR for NT (left) and HT (middle). Percentages of region lengths in mRNA sequences are reported as a reference (right). **(D)** Percentages of P-sites mapping to the three reading frames (0, 1, 2) for NT (left) and HT (right). **(E)** Meta-profiles showing the frequency of P-sites mapping at the beginning and the end of the coding sequence in NT (top) and HT (bottom). In (B), (C), (D), and (E), results are shown as the average among *n = 3* (NT) or *n* = *3* (HT) biologically independent samples, and the Standard Error of the Mean (SEM) is reported, except for (C).

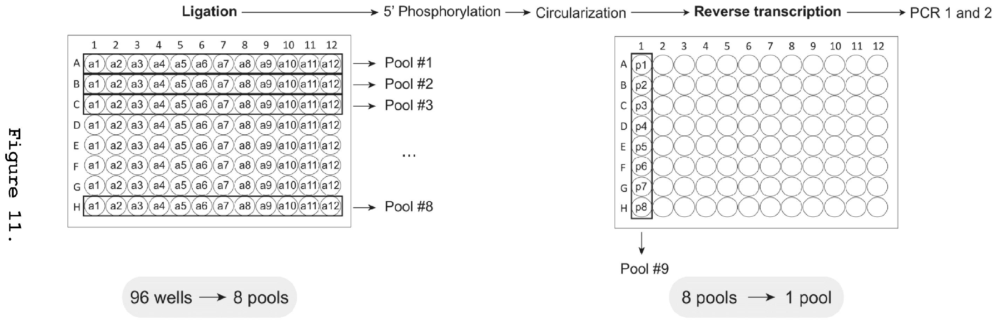

- **Figure 11****. Double-step multiplexing approach with in plate in-lysate RiboWich.** This example illustrates the use of 12 barcoded adaptors (a) and 8 barcoded RT primers (p) on a 96-well plate. In the initial step, barcoded adaptors are ligated to REFs directly in each well, enabling the pooling of 96 individual wells into 8 pools. At the RT step, each of these 8 pools is further uniquely barcoded using RT primers, allowing all samples to be combined into a single pool.

### Definitions

By "RPF" or "nude RPF" is meant a Ribosome-Protected RNA Fragment, i.e., an RNA fragment protected by the ribosome from endonuclease digestion.

By "REF" or "ribosome-embedded RNA fragment" is meant an RNA fragment protected by the ribosome from endonuclease digestion and maintained embedded within its associated ribosome.

By "adaptor-REF complex" is meant the REF ligated to the adaptor.

By "RiboSeq" is meant the name of the standard methods of ribosome profiling, which entail the extraction and gel-based size selection of RPFs prior to adaptor ligation.

By "RiboWich" is meant the name of the method herein disclosed, derived from "Ribosome sandWiched ligation".

By *"in vitro* RiboWich" is meant the library preparation method starting from the adaptor ligation to REFs after ribosome purification.

By "in-lysate RiboWich" is meant the library preparation method starting from the adaptor ligation to REFs directly in cellular lysates, without any prior purification of ribosomes.

By "in plate in-lysate RiboWich" is meant the in-lysate RiboWich library preparation method implemented in any plate format.

By "NT" and "HT" is respectively meant the cells either non-treated (NT) or treated with harringtonine (HT) .

By "active uORFs" is meant open reading frames found in the 5' untranslated region of mRNAs, upstream of the main coding sequence, which may indicate translation events according to ribosome sequencing results.

By "barcode" is meant an oligoribonucleotide sequence that enables the unequivocal identification of an individual sample among multiple samples analyzed simultaneously.

By "multiplexing" is meant the simultaneous analysis of multiple samples within a single experiment by assigning each sample a unique barcode. This approach allows all samples to be processed in a single reaction tube, significantly increasing throughput while reducing reagent consumption and technical variability.

By "P-site" is meant the specific position within either RPFs or REFs that serves as a reference for high-resolution positional analyses. Although the P-site region spans three nucleotides, only the first nucleotide is used for the analyses described in this document.

By "trinucleotide periodicity" or "periodicity" is meant the distinct pattern of P-site signal observed in ribosome profiling data that reflects how ribosomes translate the mRNAs three nucleotides at a time.

By "frame" is meant one of the three possible reading phases in which the P-site can be located along the coding sequence of mRNAs. Each frame represents a different offset (0, +1, or +2 nucleotides) from the start of the coding sequence. P-sites in frame 0 are considered to be "in-frame" or in the "correct frame" since they are located in the same reading frame as the start codon and give rise to the expected trinucleotide periodicity.

By "spacer allowing the arrest of a retrotranscriptase enzyme activity" is meant a chemical modification that can be used to mimic the presence of a naturally occurring abasic site resulting from depurination or other mechanisms. The modification involves the replacement of the deoxyribose sugar with a modified sugar molecule lacking the 2'-hydroxyl group. This modification disrupts the normal base pairing and hydrogen bonding interactions between nucleotides in the oligonucleotide. It can be selected among a 1,2'-dideoxyribose modification (dSpacer having the following chemical structure 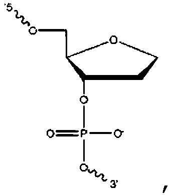 also known as abasic site), tetrahydrofuran (THF), or apurinic/apyrimidinic (AP) site. Alternatively, it can be a biotinylated blocking spacer, "Int Biotin dT", i.e., a deoxythymidine (dT) conjugated with a biotin molecule, having the following structure

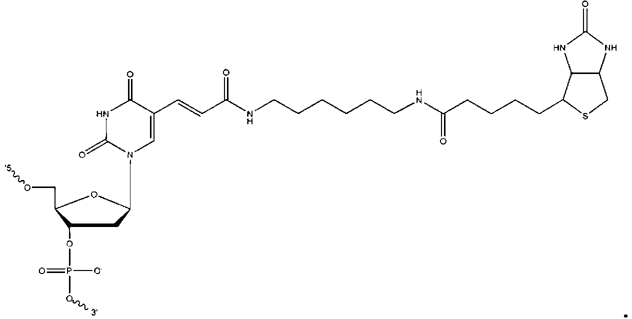

By "ribonucleotide having a modified nucleobase conferring nuclease resistance" is meant a ribonucleotide with enhanced stability and resistance against nuclease activity. Various modifications have been developed to confer nuclease resistance to ribonucleotides. These modifications can involve chemical alternations to the nucleobase structure, such as the addition of specific functional groups or substitution of certain atoms. Examples of modified nucleobases that confer nuclease resistance include, but are not limited to:
- 2'-O-Methyl (2'-OMe) Ribonucleotides: In this modification, the 2'-hydroxyl group of the ribose sugar is replaced with a methyl group. This modification enhances nuclease resistance and stability without significantly affecting RNA folding or function.
- 2'-Fluoro (2'-F) Ribonucleotides: The 2'-hydroxyl group of the ribose sugar is replaced with a fluorine atom in this modification. It improves resistance to nucleases and enhances RNA stability.
- Locked Nucleic Acids (LNAs): LNAs involve the introduction of a methylene bridge between the 2' oxygen and 4' carbon of the ribose sugar, effectively "locking" the ribose in the C3'-endo conformation. LNAs improve nuclease resistance and also enhance binding affinity to complementary RNA or DNA sequences.
- Phosphorothioate Linkages: In this modification, one of the non-bridging oxygen atoms of the phosphodiester backbone is replaced with a sulfur atom. This modification enhances nuclease resistance by introducing a chiral center and altering the conformation of the backbone.
- Peptide Nucleic Acids (PNAs): PNAs are synthetic nucleic acid analogs where the sugar-phosphate backbone is replaced with a peptide-like backbone. PNAs exhibit excellent nuclease resistance due to their nonionic nature and unique backbone structure.
- 2'-O-(2-Methoxyethyl) (2'-MOE) Ribonucleotides: This modification involves replacing the 2'-hydroxyl group with a 2-methoxyethyl group. It confers increased nuclease resistance and improves stability while maintaining RNA hybridization properties.

### Detailed description of the invention

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular solution, protocol, buffer, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular solution, protocol, buffers, or characteristic may be combined in any suitable manner in one or more embodiments.

The headings provided herein are for convenience only and do not interpret the scope or meaning of the embodiments.

The present invention is the first example of direct ligation of adaptors to ribosome-embedded RNA fragments (REFs). The RiboWich method combines at least four important advantages:
(1) simplification and acceleration of the ribosome profiling protocol;
(2) elimination of the need for size-selecting Ribosome-Protected RNA Fragments (RPFs);
(3) adaptability to multiplexing strategies offering streamlined protocols and reduced operational and resource demands;
(4) robust profiling, even with low-input samples, enabling advanced single-cell and spatial ribosome profiling applications.

The RiboWich method and kit can be used as a starting point for *in vitro* and in-lysate ribosome profiling:
a) for library preparation from purified ribosomes (suitable for ribosome profiling or selective ribosome profiling) using downstream LaceSeq^{™} from Immagina Biotechnology (LS001-12 - https://www.immaginabiotech.com/product/laceseq) ;
b) for direct in-lysate library preparation, using downstream LaceSeq^{™} from Immagina Biotechnology.

Both a) and b) can be combined with the multiplexing strategy.

It will be demonstrated that the method disclosed herein can successfully profile murine primary cells starting with tens of femtomoles of purified ribosomes, using a gel-free multiplexing approach. Furthermore, it will be shown that the same method can efficiently profile approximately a few hundred immortalized cell lines, seeded in a 96-well plate, employing the in plate in-lysate RiboWich workflow - an approach that seamlessly adapts to multiplexed, high-throughput experiments.

In one embodiment, the present invention concerns a method for ligating an adaptor to a Ribosome-embedded RNA Fragment (REF) present in a cell or tissue population, the method comprising the following steps:
(i) performing a lysis of the cell or tissue population, obtaining a lysate;
(ii) digesting the lysate with an endoribonuclease, obtaining a digested lysate;
(iii) inhibiting the endoribonuclease in the digested lysate, obtaining an inhibited lysate;
(iv) incubating the inhibited lysate with an adaptor in conditions to allow ligation of the adaptor to the REF present in the inhibited lysate, and obtaining an adaptor-REF complex, wherein the adaptor has formula (I) :

   5' OH-Nₓ-C1-L1-A_{z}-PR1-N_{y}-C2-B-OH 3' (I)

   wherein
   - N is a ribonucleotide,
   - x and y are integer numbers independently selected from 1 to 20,
   - L1 is an oligoribonucleotide sequence having a length comprised between 15 and 30 nucleotides,
   - A is an abasic site or a spacer allowing the arrest of a retrotranscriptase enzyme activity,
   - z is an integer number from 1 to 5,
   - PR1 is a first portion of a first nucleic acid domain of a sequencing platform adapter construct, having a length comprised between 10 and 80 nucleotides,
   - B is none, or a ribonucleotide having a 2'-fluoro-base, or a ribonucleotide having a modified nucleobase conferring nuclease resistance, and
   - C1 and C2 are none or a barcode sequence comprising up to 20 nucleotides, provided that at least one between C1 and C2 is none.

In one embodiment, the method further comprises a purification step (v) of the adaptor-REF complex.

In one embodiment, the purification step (v) is carried out through polysome profiling, sucrose cushion, precipitation-based techniques, or any other ribosome purification method known to the skilled man considering the common general knowledge in the technical field. These techniques are disclosed, i.a., in Lauria et al., 2020 (polysome profiling), Mehta et al, 2012, and Rivera et al., 2015 (sucrose cushion), Clamer et al., 2018, and Heiman, M., 2008 (precipitation-based techniques).

The adaptor-REF complex can be extracted and processed for the preparation of the library for the respective sequencing on a sequencing platform as disclosed i.a. in the European patent application EP-A-4506463.

The sequencing platform can be selected from those commercialized by Illumina (e.g., the HiSeg^{™}, MiSeg^{™} and NovaSeq^{™} sequencing systems); Element Bioscience (e.g., LoopSeq for AVITI^{™} sequencing systems); Singular genomics (e.g., the G4 system); Life Technologies (e.g., a SOLD sequencing system); Roche (e.g., the 454 GS FLX+ and/or GS Junior sequencing systems); MGI (e.g., E25, G400, G99, G50 and T7, T10, T20 systems). Preferably, the sequencing platform is selected from those commercialized by Illumina.

In one embodiment, the PR1 nucleic acid domain of the adaptor is derived from the N-term portion of the sequences selected from: (i) P1 adaptor by Life Technologies (as reported in "Applied Biosystems SOLiD™ 4 System Library Preparation Guide" April 2010, https://tools.thermofisher.com/content/sfs/manuals/SOLi D4_Library_Preparation_man.pdf), *(ii)* GS adaptor A by Roche (as reported in "GS FLX Titanium General Library Preparation Method Manual", April 2009, USM-00048.B, https://dna.uga.edu/wp-content/uploads/sites/51/2013/12/GS-FLX-Titanium-General-Library-Preparation-Method-Manual-Roche.pdf), *(iii)* MGI 5' adapter by MGI (as reported in "MGIEasy RNA Directional Library Prep Set User Manual", Cat. No.: 1000006385 (16 RXN) , 1000006386 (96 RXN), Kit Version: V2.1, Manual Version: 5.0) and *(iv)* SP1 sequences by Illumina (as reported in "Illumina Adapter Sequences" Document # 1000000002694 v11, May 2019; https://www.science.smith.edu/cmbs/wp-content/uploads/sites/36/2020/01/illumina-adapter-sequences-1000000002694-11.pdf) .

In one embodiment, the nucleic acid domain PR1 is derived from the N-term portion of the sequences SP1.

It is indeed evident that the expert in the field knows how to "cut and sew" the sequences of the sequencing platform adapter constructs employed by the commercially available sequencing platforms in an appropriate manner to generate the sequences of the adaptors.

Preferably, the library preparation is carried out according to the LaceSeq^{™} protocol (Immagina Biotechnology, LS001-12).

In one embodiment, the cell population is treated in a further step (0a) with an inhibitor of translation elongation to arrest ribosomes on mRNAs at their precise positions at the time of treatment before the lysis step (i).

In one embodiment, the inhibitor of translation elongation of step (0a) is selected from cycloheximide (preferably in a concentration of 10 - 200 µg/mL), and anysomicin (preferably in a concentration range of 5 - 50 µg/mL).

In one embodiment, step (0a) is carried out at a temperature of 37 °C and for a time of 2-5 minutes.

In one embodiment, the lysis step (i) is carried out using a lysis buffer comprising: a buffer solution, a divalent chloride salt, a monovalent chloride salt, a non-ionic detergent, a reducing agent, an inhibitor of translation elongation, a deoxyribonuclease, and an RNase inhibitor.

In one embodiment, the buffer solution is selected from TRIS HCl, HEPES, and phosphate buffers (preferably in a concentration range of 5-15 mM).

In one embodiment, the divalent chloride salt is MgCl₂, preferably in a concentration range of 1-5 mM for the in-lysate RiboWich approach, and 5-15 mM for the *in vitro* RiboWich approach.

In one embodiment, the monovalent chloride salt is selected from NaCl and KCl (preferably in a concentration range of 5-15 mM).

In one embodiment, the non-ionic detergent is selected from Triton X-100, Tween 20, and NP-40 (preferably in a concentration range of 0.75-1.25%).

In one embodiment, the reducing agent is dithiothreitol (DTT) (preferably in a concentration range of 0.75-1.25 mM).

In one embodiment, the inhibitor of translation elongation is selected from cycloheximide (preferably in a concentration of 10-200 µg/mL), and anysomicin (preferably in a concentration range of 5-50 µg/mL).

In one embodiment, the deoxyribonuclease is DNase I (preferably in a concentration range of 1-10 U/mL).

In one embodiment, the RNase inhibitor is selected from RiboLock RNase Inhibitor (Thermo Scientific) (preferably in a concentration of 0.1-0.5 U/µL), RNase OUT^{™} (Invitrogen) Recombinant Ribonuclease Inhibitor, RNasin Ribonuclease Inhibitor (Promega), Ribonuclease Inhibitor (New England Biolabs), or any other RNase inhibitor known to the skilled man that does not inhibit RNase I (preferably in a concentration range of 1-5 U/µL).

In one embodiment, the lysis step (i) is carried out at a temperature comprised between 0 and 4°C for at least 10 minutes with a pH comprised between 7.5-8.5.

The digestion step (ii) with an endoribonuclease is necessary for degrading the RNA molecules not associated with a ribosome leaving only REFs with a 5'-OH and a 3'-phosphate (3'P) ends that are useful for library preparation for the respective sequencing, thus avoiding improper ligation of the adaptor(s) to RNA molecules not of interest, i.e., not embedded within a ribosome.

In one embodiment, the endoribonuclease is selected from RNase I (preferably in a concentration range of 0.1-0.4 U/µL), Micrococcal Nuclease (MNase), and any other endonuclease that hydrolyzes single-stranded RNA to nucleoside 3'-monophosphates or cyclic phosphates known to the skilled man.

In one embodiment, the digestion step (ii) is carried out either (a) at a temperature comprised between 20 and 25°C, with an incubation comprised between 30 and 60 minutes, in a thermoshaker with shaking at 300-600 rpm, or (b) at a temperature of about 37°C, with an incubation comprised between 5 and 20 minutes, in a thermoshaker with shaking at 300-600 rpm.

In one embodiment, the endoribonuclease inhibition step (iii) is carried out using the endonuclease inhibitor SUPERase·In^{™} RNase Inhibitor (Invitrogen^{™}) (preferably in a concentration range of 0.1-1 U/µL), or by freezing the digested lysate (preferably for at least 30 minutes at -80°C). Alternatively, the endoribonuclease inhibition step (iii) is carried out by any method known to the skilled man in view of the common general knowledge of the filed that ensures efficient endonuclease inhibition in the digested lysate while preserving ribosome integrity.

In one embodiment, the endoribonuclease inhibition step (iii) by treatment with SUPERase·In^{™} RNase Inhibitor (Invitrogen^{™}) is carried out either (a) at 37°C temperature with an incubation comprised between 5 and 20 minutes, in a thermoshaker with shaking at 300-600 rpm, or (b) at a temperature comprised between 0 and 4°C for at least 10 minutes.

In one embodiment, the endoribonuclease inhibition step (iii) by freezing the digested lysate is carried out at -80°C for at least 30 minutes.

In one embodiment, when the method is carried out in well plates, the steps (ii) and (iii) can be combined into a single step, and carried out at a temperature comprised between 20-25°C and an incubation time between 30 and 60 minutes, in a thermoshaker with shaking at 300-600 rpm.

In one embodiment, before the incubation step (iv), a ribosome isolation step (iiia) can be carried out, thus allowing ribosome profiling or selective ribosome profiling.

In one embodiment, the ribosome isolation step (iiia) is carried out by: sucrose gradients, polysome profiling, and purification-based techniques such as agarose beads, agarose-acrylamide beads, cellulose beads, iron oxide beads, graphene beads, silica or silica mixture beads, polystyrene beads, polycarbonate beads, polypropylene beads, and/or combinations thereof.

In one embodiment, when a ribosome isolation step (iiia) has been carried out, the ligation step (iv) is performed using a ligation buffer comprising: a buffer solution, a divalent chloride salt, a monovalent chloride salt, a reducing agent, an inhibitor of translation elongation, an RNase inhibitor, a ribonucleotide triphosphate, a divalent cation cofactor, an RNA ligation enzyme, and an adaptor of formula (I).

In one embodiment, when bypassing the ribosome isolation (iiia), the ligation buffer further comprises: a non-ionic detergent and a deoxyribonuclease.

In one embodiment, the buffer solution is selected from TRIS HCl, HEPES, and phosphate buffers (preferably in a concentration range of 5-100 mM).

In one embodiment, the divalent chloride salt is MgCl₂ (preferably in a concentration range of 1-5 mM).

In one embodiment, the monovalent chloride salt is selected from NaCl and KCl (preferably in a concentration range of 5-15 mM, or - if a step (iiia) has been carried out - in a concentration range of 5-100 mM.

In one embodiment, the reducing agent is dithiothreitol (DTT) (preferably in a concentration range 5-20 mM, or - if a step (iiia) has been carried out - in a concentration range 1-20 mM).

In one embodiment, the inhibitor of translation elongation is selected from cycloheximide (preferably in a concentration of 10-200 µg/mL), and anysomicin (preferably in a concentration range of 5-50 µg/mL).

In one embodiment, the RNase inhibitor is selected from RiboLock RNase Inhibitor (Thermo Scientific) (preferably in a concentration of 0.1-0.5 U/µL), RNase OUT^{™} (Invitrogen) Recombinant Ribonuclease Inhibitor, RNasin Ribonuclease Inhibitor (Promega), Ribonuclease Inhibitor (New England Biolabs), or any other RNase inhibitor that does not inhibit RNase I known to skilled man (preferably in a concentration range of 1-5 U/µL).

In one embodiment, the ribonucleotide triphosphate is guanosine-5'-triphosphate (GTP) (preferably in a concentration range of 0.05-0.4 mM).

In one embodiment, the divalent cation cofactor is MnCl₂ (preferably in a concentration range of 0.5-4 mM).

In one embodiment, the RNA ligation enzyme is RtcB ligase (New England Biolabs) (preferably in a concentration range of 0.5-2 µL/ 50 µL of reaction).

In one embodiment, the adaptor of formula (I) is present in a concentration range of 0.01 - 0.04 µM or - if a step (iiia) has been carried out - in a concentration range of 0.1 nM - 0.01 µM.

In one embodiment, the non-ionic detergent is selected from Triton X-100, Tween 20, and NP-40 (preferably in a concentration range of 0.75-1.25%).

In one embodiment, the deoxyribonuclease is DNase I (preferably in a concentration range of 1-10 U/mL).

In one embodiment, the ligation step (iv) is carried out at 37°C for at least 1 hour with a pH comprised between 7.5-8.5, in a thermoshaker with shaking at 300-600 rpm.

In one embodiment, when more than one cell population is employed, the adaptor-REF complexes of each cell population obtained at the end of the ligation step (iv) are pooled together for multiplexing sequencing, wherein the domains C1 or C2 of formula (I) have a nucleotide sequence different for each cell population. In such a case, each adaptor-REF complex from a specific cell population is provided with a barcode specific for the cell population.

In one embodiment, the cell population contains less than 1 femtomoles of ribosomes.

In one embodiment, before the lysis step (i), the cell population is treated in a further step (0b) with an inhibitor of translation initiation that arrests ribosomes only at translation start sites. This ensures that the majority of REFs align with translation start sites, enabling the identification of annotated coding regions as well as the discovery of novel, unannotated ORFs.

In one embodiment, the inhibitor of translation initiation of step (0b) is harringtonine, preferably in a concentration range of 1-2 µg/mL.

In one embodiment, the cell population treatment with harringtonine I is carried out at 37°C, with an incubation comprised between 1 and 10 minutes.

In one embodiment, the present invention concerns a kit suitable for implementing the above-described method for ligating an adaptor to a REF, the kit comprising:
(a) an adaptor of formula (I)

   5' OH-Nₓ-C1-L1-A_{z}-PR1-N_{y}-C2-B-OH 3' (I)

   wherein
   - N is a ribonucleotide,
   - x and y are integer numbers independently selected from 1 to 20,
   - L1 is an oligoribonucleotide sequence having a length comprised between 15 and 30 nucleotides,
   - A is an abasic site or a spacer allowing the arrest of a retrotranscriptase enzyme activity,
   - z is an integer number from 1 to 5,
   - PR1 is a first portion of a first nucleic acid domain of a sequencing platform adapter construct, having a length comprised between 10 and 80 nucleotides,
   - B is none, or a ribonucleotide having a 2'-fluoro-base, or a ribonucleotide having a modified nucleobase conferring nuclease resistance, and
   - C1 and C2 are none or a barcode sequence comprising up to 20 nucleotides, provided that at least one between C1 and C2 is none;
(b) an endonuclease enzyme;
(c) an inhibitor of the endonuclease enzyme; and
(d) a ligase enzyme.

In one embodiment, the PR1 nucleic acid domain is derived from the N-term portion of the sequences selected from: *(i)* P1 adaptor, *(ii)* GS adaptor A, *(iii)* MGI 5' adapter, and *(iv)* SP1 sequences.

In one embodiment, the endonuclease enzyme is an endoribonuclease enzyme suitable to hydrolyze single-stranded RNA into nucleoside 3'-monophosphate or cyclic phosphate.

In one embodiment, the endonuclease enzyme is selected from RNase I and MNase.

In one embodiment, the endonuclease inhibitor is SUPERase · In^{™} RNase inhibitor.

In one embodiment, the ligase enzyme is an RNA ligase enzyme.

In one embodiment, the ligase enzyme is RtcB ligase.

Over the past 15 years, ribosome profiling has revolutionized our ability to investigate protein synthesis across a wide range of biological samples (Ingolia et al. 2009). Yet, despite its widespread use, traditional ribosome profiling methods face several limitations that often result in mediocre data quality (Wang and Mao, 2023). Such limitations include i) timeconsuming protocols, ii) difficulties in obtaining high-quality data from challenging, low-input samples, and iii) inefficient detection of less frequent non-canonical translational events (Wang and Mao, 2023).

To overcome these challenges, the present inventors developed RiboWich (derived from Ribosome sandWiched ligation), a first-of-its-kind technology that enables the ligation of sequencing adaptors to . This innovative and unique approach bypasses the need for RNA extraction, RPF size selection, and purification prior to adaptor ligation, simplifying and accelerating the entire workflow. RiboWich effectively captures authentic fragments of the expected 30 to 34 nt lengths, enriched within the coding sequence in the correct reading frame, providing robust ribosome positional information. These findings underscore the strength and reliability of RiboWich as a valuable method for ribosome profiling, offering enhanced efficiency and data quality for studying protein synthesis.

RiboWich is uniquely suited for multiplexing experiments, as the ligation occurs directly to REFs before any additional processing. By incorporating unique barcodes at this early stage along the protocol, individual samples can be pooled after a single reaction and then processed together in a single tube throughout all further library preparation steps. The approach streamlines sample handling and reduces the time and costs typically associated with processing each sample independently. RiboWich can be implemented in a dualstep multiplexing strategy, further increasing the number of samples that can be processed in parallel in a single experiment. The approach consists of incorporating barcoded reverse transcription (RT) primers, introducing an additional layer of multiplexing **(****Figure 11****).** With this strategy, the number of uniquely barcoded samples in one single experiment is determined by the equation *a x p,* where a is the number of ligation barcoded adaptors and *p* is the number of RT barcoded primers. This double-step multiplexing approach is particularly advantageous when in-lysate RiboWich is performed in plate (in plate in-lysate RiboWich), as it further enhances the scalability and experimental throughput. In particular, this approach makes RiboWich highly adaptable to any plate format, with the only limitation being the number of available a and *p* barcoded sequences.

RiboWich not only enhances the throughput of the ribosome profiling workflow but also expands its applicability to challenging low-input samples by minimizing material loss throughout the entire process. While recent studies have made great strides toward low-input ribosome profiling, generating libraries from as few as thousands of cells (Hornstein, N. et al., 2016; Xiong et al., 2022; Li et al., 2022; Zhang et al.,2022; Meindl et al. 2023, Froberg et al.,2023; Ozadam et al., 2023; summarized in **Table 1**) or from single-cells (VanInsberghe et al., 2021), these methods come with specific limitations tied to the library construction itself. These techniques rely on the template-switch and the dual-ligation method, which, while powerful, have sequence biases and are not compatible with early-stage barcode integration, making them unsuitable for multiplexing (Wang and Mao., 2023). In contrast, RiboWich combines the use of tailored multiplexed adaptors with a gel-free protocol, streamlining the workflow while enabling high-resolution profiling.

The non-optimal signal-to-noise ratio often observed in RiboSeq experiments hampers the detection and analysis of non-canonical translational events (Mao and Qian, 2023), which, despite occurring at low frequency, play crucial roles in nuanced translational regulation, particularly in stress responses (Khitun et al., 2019; Moro et al., 2021), cellular differentiation (Vatikioti et al., 2019), and various disease contexts (Silva et al., 2019). The present disclosure demonstrates that RiboWich detects a significantly greater number of active uORFs that reveal ribosomes with a stronger periodicity accumulating at the translation start site, thus surpassing the capabilities of the standard ribosome profiling method. Moreover, it has been observed that RiboWich captures a higher number of uORFs associated with detected peptides (Mudge et al., 2022) compared to RiboSeq. These results provide strong evidence that RiboWich unravels active translation occurring in non-canonical ORFs, offering a more comprehensive and accurate approach to studying translation regulation. Changing the cellular context, and particularly investigating primary murine cortical neurons and astrocytes, RiboWich identified CNs- and astrocytes-specific uORFs that allow an accurate dissection of translation differences between the two cell types.

In conclusion, RiboWich enhances ribosome profiling by streamlining the workflow, improving data quality, and enabling low-input ribosome profiling. With its early barcoding step and efficient ligation, RiboWich easily adapts to a multiplexing approach, reduces protocol complexity, and better captures non-canonical events like uORFs, essential for understanding translation regulation. Taken together, RiboWich serves as a pillar for enabling the development of future RiboSeq methods and technologies.

**Table 1**

| **Reference** | **Applications** | **# of cells** | **Total RNA (ng)** | **RPFs/REFs (pg)** | **Ribosomes (pmol)** |
|---|---|---|---|---|---|
| Hornstein, N. et al., 2016 | Low-input | high-input samples (5mg brain tissue) | 100* | **1000**** | 0.1 |
| Meindl A. et al., 2023 | Low-input | high-input samples (immortalized and yeast cells) | 200* | **1000**** | 0.1 |
| Zhang C. et al., 2022 | Low-input and single-cell | 5,000 mESCs | 0.08* | 400* | 0.04 |
| | | 1 mouse oocytes | | | |
| **This study** | **Multiplexed low-input** | **2,000 immortalized cells** | **20-40*** | **160*** | **0.016*** |
| Li et al., 2022 | Low-input | 1,000 immortalized cells | 10/20** | 80* | 0.008 |
| | | 1,000 primary cells | 50/100** | | |
| Froberg J. E. et al., 2023 | Low-input | 1,000 immortalized cells | 16* | 80* | 0.008 |
| | | 2,500 primary cells | | | |
| Xiong Z. et al., 2022 | Low-input and single-cell | 50 immortalized cells | 0.8* | 4* | 0.0004 |
| | | 1 mouse oocyte | | | |
| Ozadam H. et al., 2023 | Low-input and single-cell | 1-100 immortalized cells | 0.016 - 1. 6* | 0.08 - 8* | 8 10⁻⁶ - 0.0008 |
| | | 1 mouse oocyte | | | |

| | | | | | |
|---|---|---|---|---|---|
| * Estimated; ** Measured | | | | | |

**Figure 1** shows the steps of the RiboSeq and RiboWich protocols. Both protocols start treating cells with cycloheximide (step a), an inhibitor of translation elongation that stabilizes ribosomes on mRNAs. Step b consists of a washing step with PBS in the case of RiboSeq and the Preserving Solution in the case of RiboWich. In step c, cells are collected and subsequently lysed (step d) with Standard Cytoplasmatic Lysis Buffer (RiboSeq) and RiboWich Cytoplasmatic Lysis Buffer (RiboWich). In step e, lysates are treated with RNase, which digests the free RNA, leaving only the mRNA fragments protected by the ribosomes. Upon RNase incubation, its action is stopped by adding an inhibitor (step f). For RiboSeq, in steps g and h, ribosomes are isolated, RNA is extracted, and RPFs are size-selected and purified. The library preparation typically starts with an end repair reaction (step i), followed by the adaptor incorporation (step j). In step k, the final steps of library preparation are carried out, differing based on the chosen protocol. In RiboWich, upon RNase inhibition, two approaches are available: (1) the *in vitro* method and (2) the in-lysate method. For *in vitro* RiboWich, ribosomes are isolated and resuspended in the *in vitro* RiboWich Ligation Solution to allow the adaptor to ligate the REFs within the purified ribosomes (step j). For the in-lysate RiboWich, a purification step is not required. Indeed, REFs within ribosomes (and the residual, unpurified lysate context) undergo the adaptor ligation reaction in the in-lysate RiboWich Ligation Solution (step j). Eventually, in step k, the final steps of library preparation are carried out following the LaceSeq^{™} protocol described by Immagina Biotechnology.

### RESULTS

### RiboWich captures bona fide RNA fragments in HEK293T

A key step in RiboSeq experiments is the generation and isolation of Ribosome-Protected Fragments (RPFs - **Figure 2A****, left).** RPFs generation involves the treatment of cytoplasmatic lysates with an endonuclease, most commonly RNase I, that degrades the RNA not protected by ribosomes, leaving ribosome footprints with a 5'-hydroxyl (5' OH) and a 3-phosphate (3'P) end that are used for library preparation. With the aim of developing innovative solutions for applying RiboSeq to multiplexed experimental designs and robust single-cell and spatial profiling, the inventors worked on optimizing the key limiting step of the size selection and purification of RPFs. This step has remained unchanged since RiboSeq's introduction in 2009 but represents a major bottleneck for developing robust technologies and rapid protocols for high-resolution ribosome profiling.

Leveraging the enzymatic capabilities of RtcB ligase to directly ligate RNA fragments with a 3'-phospate end (3'-P) to convenient adaptors (using the commercial kit named LaceSeq^{™}), the inventors developed RiboWich, a streamlined solution that enables direct adaptor ligation to RNA fragments while they are still embedded within ribosomes **(****Figure 2A****, right).** Upon adaptor ligation, the remaining steps of LaceSeq^{™} library preparation are performed, according to manufacturer instructions, to generate a dsDNA library suitable for sequencing on the Illumina platform. By performing the adaptor ligation as the very first reaction, which occurs before RNA extraction, RPFs isolation, or any other library preparation reaction, RiboWich seamlessly integrates with multiplexing strategies **(****Figure 2B****).** Specifically, after the ligation of distinct barcoded adaptors, samples can be pooled and processed together in the following steps of the protocol. This approach enables multiplexed RiboSeq, with the number of parallel samples being determined by the availability of unique adaptors. Pooling multiple barcoded samples in a single tube not only streamlines further steps of library preparation but also drastically reduces the input material required.

To demonstrate the effectiveness of the ligation of the adaptor to REFs and show that RPFs purification is not essential for ribosome profiling, RiboWich was compared to a standard method (hereafter referred to as RiboSeq) **(****Figure 3A****)** which includes gel-based size selection of RPFs, and end-repair of RNA fragments prior to adaptor ligation. Specifically, the LaceSeq^{™} protocol developed by Immagina Biotechnology was used for library preparation in the RiboSeq workflow. Briefly, for RiboSeq, the RNA was extracted from isolated ribosomes obtained from RNase I-treated cytoplasmatic lysates **(****Figure 3B****).** Next, size-selected RPFs (28-32 nt) were purified from denaturing gel **(****Figure 3C****)** and the library was prepared following the LaceSeq^{™} canonical steps. As proof of principle, we applied in parallel the RiboWich protocol using ribosomes with embedded RNA fragments purified from HEK293T lysates after RNase I treatment and subsequent inhibition **(****Figure 3D****).** After resuspending the ribosomes in an optimized *in vitro* Ribosome Solution, we ligated the adaptor to REFs. For consistency and to enable a direct comparison, we here introduced a gel-based purification step of the ligation products (80 nt) **(****Figure 3E****).** Next, we proceeded with the remaining steps of library preparation following the LaceSeq^{™} protocol. Notably, in both cases we obtained a library detected at 200 bp, suggesting that the ligation of the adaptor to REFs is likely to occur **(****Figure 3F** and **G****).**

After sequencing, we performed a comprehensive quantitative assessment of both RiboSeq and RiboWich datasets, analyzing various performance indicators as quality measures (Lauria et al., 2018). First, the percentage distribution of reads in the preprocessing and alignment categories **(Table 2)** shows that RiboWich captures more reads aligning to the transcriptome (mean = 16.5%) compared to RiboSeq (mean = 12.6%) **(****Figure 4A****).** The mean percentage distribution of read lengths peaks at the expected RPFs dimension (30-34 nt) in both methods **(****Figure 4B****).** RPFs are expected to be predominantly located within the coding sequence (CDS) of mRNAs, where the P-sites, taken as a reference position, are expected to exhibit trinucleotide periodicity. To assess the enrichment of ribosome signals in the CDS, we analyzed the percentage of P-sites mapping on the 5' untranslated region (UTR), CDS, and 3' UTR portions of mRNAs for RiboSeq and RiboWich **(****Figure 4C****, left** and **middle),** compared to a uniform distribution weighted on region lengths, and simulating random P-site positioning along mRNAs **(****Figure 4C****, right).** In RiboSeq and RiboWich, the percentage of P-sites mapping on each mRNA region is similar, showing a strong enrichment in the CDS. In addition, the majority of the P-sites in the CDS fall in the correct reading frame to a similar extent in both methods **(****Figure 4D****).** Next, we visually inspected the trinucleotide periodicity of P-sites mapping along the CDS, around the start and stop codons, also including portions of the UTRs. As expected for high-quality ribosome profiling data, both RiboSeq and RiboWich show sharp peaks associated with periodicity along the CDS and low signal on the UTRs **(****Figure 4E****),** demonstrating the success of the ligation to REFs. Importantly, a direct comparison of the ribosome occupancy expressed as Reads Per Kilobase of transcript per Million mapped reads (RPKM) shows a strong correlation between the methods **(****Figure 4F****).** To further reinforce our findings, we performed Gene Ontology enrichment analysis of mRNAs captured by both methods and found similar results **(****Figure 4G****).** Overall, these results demonstrate that RiboWich performs at least as efficiently as established ribosome profiling protocols and is capable of capturing *bona fide* RNA fragments providing positional information of ribosomes across the translatome.

To explore RiboWich's potential and sensitivity, we performed a translation-initiation sequencing experiment designed to investigate non-canonical translation events. These are interesting case studies, as upstream ORFs (uORFs), though present in 40-50% of mammalian transcripts, remain largely unannotated, and their translational status is still poorly understood. Existing ribosome profiling methods has been considered not sensitive enough to capture these events with high probability and resolution (Wang and Mao., 2023), and proteomics methods often struggle to identify short, unstable peptides derived from non-canonical events. These limitations underscore the importance of high-quality RiboSeq data to accurately distinguish novel uORFs from background noise, making this an excellent test case for showcasing RiboWich's performance compared to RiboSeq.

We applied RiboSeq and RiboWich in HEK293T treated with harringtonine (harringtonine-treated, HT, **Figure 5A-C****),** a molecule that inhibits translation initiation and stalls ribosomes at translation start sites. This ensures that most RPFs map to the translation start sites across the transcriptome, allowing both the identification of annotated coding regions and the discovery of novel, unannotated ORFs. In the HT datasets, we observed that RiboWich shows a higher percentage of reads aligned to the transcriptome (mean = 6.02%) compared to RiboSeq (mean = 3.52%) **(Table 2,** **Figure 5D****).** The mean percentage distribution of read lengths peaks at the expected RPFs dimensions (30-34 nt) for both methods **(****Figure 5E****),** and the majority of P-sites in the CDS fall in the correct reading frame **(****Figure 5F****).** The trinucleotide periodicity of the P-sites across the meta-profiles shows a robust increase in the P-sites frequency at the start codon and a relative decrease along the CDS in both RiboSeq and RiboWich datasets **(****Figure 5G****),** suggesting that our method can successfully detect positional changes.

Both RiboSeq and RiboWich show an enrichment of P-sites in the CDS, with respect to 5' and 3' UTRs **(****Figure 6A****).** While the percentage of P-sites mapping on the 5' UTR is generally high, implying the presence of non-canonical initiation events, RiboWich exhibits a significant enrichment in the 5' region compared to RiboSeq **(****Figure 6A****).** This result suggests that RiboWich is likely more effective than RiboSeq at capturing non-canonical translation events occurring in the 5' UTR. To dissect whether RiboWich could aid in studying these alternative events, we developed and implemented in R a dedicated computational data filtering pipeline **(****Figure 6B****).** Initially, our workflow identifies all potential uORFs in the 5' UTR, characterized by a start codon and a stop codon in frame with respect to the start site. Then, to minimize false positives and accurately select only actively translating putative uORFs, our pipeline applies three sequential filters. First, uORFs with more than 10 RPKM are retained. Second, uORFs exhibiting more than 25% of the reads mapping at the start codon in the HT condition, as expected due to ribosome accumulation at initiation sites, are selected. Third, the uORFs displaying trinucleotide periodicity in NT samples are kept. By intersecting the resulting uORFs with the RibouORF database (Liu, et al., 2023), we identified 502 active uORFs in RiboSeq and 601 in RiboWich. Interestingly, in RiboWich, the number of uORFs, normalized by the number of canonical ORFs, is significantly higher compared to RiboSeq **(****Figure 6C****),** highlighting RiboWich's superior ability to capture active uORFs compared to standard RiboSeq. The detected active uORFs displayed robust trinucleotide periodicity in both NT and HT conditions and methods. Importantly, RiboWich shows a significantly higher in-frame P-site signal compared to RiboSeq **(****Figure 6D****),** a more distinct trinucleotide periodicity in the meta-profiles around the translation initiation region in the NT condition **(****Figure 6E****, top),** and a clearer peak at the start codon in the HT samples **(****Figure 6E****, bottom).** Finally, the accuracy of individual uORF detection was assessed by intersecting our datasets with a list of uORFs producing peptides identified in proteomic datasets. Remarkably, we observed that RiboWich captures a higher number of uORFs associated with detected peptides compared to RiboSeq **(****Figure 6F****),** confirming that our innovative technology outperforms existing RiboSeq protocols in capturing active uORFs, with high-quality and at single-nucleotide resolution.

**Table 2**

| **RiboSeq Type** | **Condition** | **Replicate** | **Initial reads (N x 10^6)** | **Untrimmed (%)** | **rRNA (%)** | **tRNA (%)** | **Transcriptome (%)** | **Transcriptome (N x 10^6)** | **Unmapped (%)** |
|---|---|---|---|---|---|---|---|---|---|
| RiboSeq | Control | 1 | 20.28 | 0.29 | 75.80 | 5.50 | 16.10 | 3.27 | 0.02 |
| | | 2 | 6.26 | 0.20 | 83.80 | 3.80 | 10.60 | 0.66 | 0.02 |
| | | 3 | 21.54 | 0.35 | 81.70 | 5.10 | 11.00 | 2.36 | 0.02 |
| | Harringto nine | 1 | 365 | 1.21 | 87.30 | 5.90 | 4.20 | 15.43 | 0.01 |
| | | 2 | 4.39 | 0.26 | 90.90 | 5.00 | 2.70 | 0.11 | 0.01 |
| | | 3 | 16.72 | 0.33 | 85.90 | 8.40 | 3.70 | 0.61 | 1.73 |
| RiboWich | Control | 1 | 33 | 0.66 | 73.80 | 1.10 | 18.60 | 6.04 | 0.06 |
| | | 2 | 27 | 0.68 | 79.10 | 1.10 | 15.20 | 4.08 | 0.04 |
| | | 3 | 66 | 0.67 | 78.10 | 1.40 | 15.70 | 10.31 | 0.04 |
| | Harringto nine | 1 | 30 | 0.66 | 89.30 | 1.90 | 5.00 | 1.48 | 0.03 |
| | | 2 | 55 | 0.54 | 89.30 | 1.30 | 5.50 | 1.48 | 0.03 |
| | | 3 | 45 | 0.49 | 86.30 | 2.30 | 7.60 | 1.48 | 0.03 |

### Multiplexed RiboWich enables robust profiling in low-input samples

To expand on these findings and explore RiboWich in low-input samples, we prepared libraries using 25 femtomoles of purified ribosomes from NT and HT primary cortical neurons (CNs) and astrocytes from wild-type C57BL/6 mice. Usually, these samples pose significant challenges due to the low extraction yield of macromolecules, specifically ribosomes and RPFs. To optimize the processing of low-input samples, we enhanced the RiboWich workflow in two key areas. First, as proof of principle, we introduced multiplexed adaptors for barcoding REFs, enabling the simultaneous processing of up to six distinct samples. Following the ligation, the samples were pooled into a single tube, reducing sample loss and improving the efficiency of downstream enzymatic steps **(****Figure 7A****).** Second, we developed a gel-free library preparation protocol, eliminating the need for gel-based purification and significantly reducing the risk of material loss.

The distribution of read lengths peaks within the expected range of RPF lengths (30-36 nt) for both cell types and in both conditions **(****Figure 7B** and **Figure 8A****).** For both cell types in NT conditions, the majority of P-sites map to the CDS, with lower percentages mapping to the 5' and 3' UTRs **(****Figure 7C****).** The HT samples display a higher percentage of P-sites mapping at the 5' UTR **(****Figure 8B****),** further supporting the hypothesis that RiboWich captures non-canonical translation events. Next, we verified the trinucleotide periodicity of ribosome P-sites in the CDS of NT and HT samples **(****Figure 7D-E****,** and **Figure 8C-D****).** In line with the harringtonine function, the HT samples show a relative increase in the signal at the start codon and a decrease along the CDS **(****Figure 8D****).** Prompted by the observation that RiboWich captures *bona fide* RNA fragments in both cell types, we investigated the presence of active uORFs in CNs and astrocytes following the previously described approach **(****Figure 6B****).** We identified 1,343 CNs-specific and 23 astrocyte-specific active uORFs, and 42 active uORFs shared between the two cell types **(****Figure 7F****).** Interestingly, the active uORF of Prkcz, included among the CNs-specific uORFs, is known to be associated with peptides detected in proteomics data (Mudge et al., 2022) and regulates the translation of its canonical ORF **(****Figure 7G****).** The results support our previous findings in HEK293T cells and showcase the key role of multiplexing the protocol, which opens new opportunities for future technological applications.

### Multiplexed in-lysate RiboWich: direct ligation to REFs in cellular lysates

RiboWich stands out due to its unique ability to perform ligation directly to REFs, setting it apart from all existing conventional approaches. Building on this strong foundation, we expand RiboWich's experimental applications and enhance the versatility of ribosome profiling. To further streamline and accelerate the workflow, we developed a refined version of RiboWich, named in-lysate RiboWich. This innovative approach eliminates the need for ribosome purification entirely by enabling the direct ligation of adaptors to REFswithin cytoplasmic lysates without any prior isolation steps, becoming the only RiboSeq method that completely avoids ribosome isolation and RPF extraction before adaptor ligation **(****Figure 9A****).** Additionally, by introducing barcoded multiplexed adaptors in the cellular lysates, the samples can be pooled for further isolation and extraction of ligated REFs in a single reaction **(****Figure 9B****).** This approach is particularly beneficial for experiments involving a low number of cells per sample, where separate RPFs or REFs extraction from each sample would be inefficient and would yield insufficient RNA for subsequent library preparation. In the case of high-throughput RiboSeq applications, uniquely barcoded adaptors can be ligated in the cellular lysates within multi-well plates. Soon after the in-lysate adaptor ligation, samples from each well can be pooled to proceed with library preparation in a single reaction tube **(****Figure 9B****).**

We tested multiplexed in-lysate RiboWich using 1 10⁵ HEK293T cells per sample. In particular, we used three NT control samples and three HT samples, for a total number of six samples. After cellular lysis and RNA digestion, we performed the ligation of barcoded adaptors to REFs in the cellular lysate of each sample, separately. Next, we pooled the six samples together and proceeded with RNA extraction and library preparation according to the LaceSeq^{™} protocol. Finally, we proceeded with library sequencing and analyzed the data as previously described. The large percentage of reads mapping to the CDS confirms that we are capturing *bona fide* RPFs **(****Figure 9C****),** similar to what was observed for the *in vitro* RiboWich samples. By analyzing the percentage of P-sites falling in the CDS across the three possible reading frames (0,1, or 2), we observed that the majority of P-sites is in the correct reading frame (0) in both NT and HT samples **(****Figure 9D****, top and bottom,** respectively). Lastly, the meta-profile of NT samples shows peaks associated with trinucleotide periodicity along the CDS, around the start and stop codons **(****Figure 9E****, top).** The meta-profile of HT samples shows an increase in the P-sites density at the start codon and a relative decrease along the CDS, as expected **(****Figure 9E****, bottom)** . Moreover, in both NT and HT samples, a lower P-sites density is observed in the UTRs with respect to the CDS.

Overall, in-lysate RiboWich represents a major step forward in making ribosome profiling more accessible, efficient, and versatile for diverse experimental needs.

Next, to reduce the number of starting cells required, we adapted the in-lysate RiboWich workflow to a 96-well plate format **(****Figure 10A****).** Briefly, cells were treated with cycloheximide (step a), washed with the Preserving Solution (step b), and lysed with the RiboWich Cytoplasmatic Lysis Buffer supplemented with RNase I (step c). Lysis and RNase I digestion were combined into a single step to streamline and optimize the protocol for plate-based applications. Step d involved the RNase inhibition and, next, the adaptor ligation to REFs occurred (step e). Upon ligation, barcoded samples were pooled together in one single tube. The library preparation (step f), sequencing, and data analysis were conducted as previously described. Libraries from 1 femptomoles of ribosomes, corresponding to around 2 10⁴ HEK293T cells per sample, were obtained in three NT and three HT replicates. Read length distributions peak within the expected range (30-36 nt) for both NT and HT conditions **(****Figure 10B****).** Most P-sites map to the CDS, with a smaller proportion mapping to the 5' and 3' UTRs **(****Figure 10C****).** As previously observed, HT samples exhibit a higher percentage of P-sites mapping to the 5' UTR. The trinucleotide periodicity of ribosome P-sites in the CDS shows good quality in both NT and HT samples **(****Figure 10D-E****),** demonstrating that the in plate in-lysate RiboWich is successful, opening the unique opportunity to scale down the starting amount of cells and allowing a high-throughput ribosome profiling.

### MATERIALS AND METHODS

### Cell lines

HEK293T cell line was maintained in culture in Dulbecco's Modified Eagle's Medium (DMEM, Euroclone), 10% FBS (GIBCO, Life Technologies), 2 mM L-Glutamine (GIBCO, Life Technologies), and 1% penicillin/streptomycin (GIBCO, Life Technologies) at 37°C in a 5% CO₂ atmosphere.

### Wild-type C57BL/6 mice for primary cortical neurons and astrocytes cultures

Wild-type C57BL/6 mice were obtained from Charles River and were used to prepare primary cortical neurons and astrocytes cultures. Animal care and experimental procedures were performed in accordance with the Ethical Committee of the University of Trento and were approved by the Italian Ministry of Health (576/2021-PR). Animals were maintained with access to food and water ad libitum and kept at a constant temperature (19-22°C) with a 12:12 h light/dark cycle.

### Cultures of primary cortical neurons from wild-type C57BL/6 mice

Primary cortical neurons were obtained from wild-type mice at embryonic stage E15.5 as previously described (Migazzi et al., 2021). Meninges were removed from cortices and were digested in papain solution (20U papain, 500 µM EDTA, 100 µM Cystine, 26 mM Sodium Bicarbonate in 1X Earle's Balanced Salt Solution (EBSS, Gibco) at 37°C for 20 minutes. After DNase I treatment at 37°C for 3 minutes, they were centrifuged at 1578 g for 5 minutes. The pellet was resuspended in EBSS supplemented with Bovine Serum Albumin and Trypsin Inhibitor. Cells were centrifuged at 1578 g for 10 minutes and resuspended in the plating medium (10% FBS, 1% penicillin/streptomycin in MEM with L-Glutamine). Cortical neurons were seeded on pre-coated wells with poly-D-lysine (Sigma). The day after the whole medium was replaced with Neurobasal medium added with 1% B27, 1 mM Sodium Pyruvate, 1% penicillin/streptomycin, 2 mM L-Glutamine, and 8 µM AraC (Sigma). Cortical neurons were cultured at 37°C with 5% CO₂.

### Cultures of astrocytes from wild-type C57BL/6 mice

Primary astrocytic cells were cultured from wild-type newborn pups (P0-P2). After meninges removal, cortical tissues were enzymatically digested in papain solution and treated with DNase I, following the same procedure as for neuronal culture. After digestion blockage, cells were resuspended in DMEM (Gibco) supplemented with 10% FBS, 2 mM L-Glutamine, and 1% penicillin/streptomycin and plated. At DIV1, the medium was replaced with fresh complete medium. Cells were cultured at 37°C with 5% CO₂.

### RiboSeq protocol

Cytoplasmatic lysates were prepared according to (Tebaldi et al., 2012). Briefly, cells were seeded in 10 cm diameter dishes and grown until they reached 70-80% confluence. Specifically for the translation initiation sequencing, cells were treated for 10 minutes with 2 µg/mL harringtonine (Abcam, ab141941-10) at 37°C. Then, not-treated (NT) and harringtonine-treated (HT) cells were incubated for 3 minutes and 30 seconds at 37°C with cycloheximide (10 µg/mL) (Sigma Aldrich, C7698), an inhibitor of translation elongation that was added to the culture medium to stabilize ribosomes on mRNAs. Upon incubation, dishes were put on ice and washed three times with cold PBS (Gibco, Life Technologies) supplemented with 10 µg/mL cycloheximide. Next, 100-300 µL of the Standard Cytoplasmatic Lysis Buffer (10 mM Tris-HCl pH 7.5, 10 mM MgCl₂, 10 mM NaCl, 1% Triton X-100, 5 U/mL DNase I (Thermo Scientific), 0.2 U/µL RiboLock RNase Inhibitor (Thermo Scientific), 1 mM DTT (Sigma Aldrich), 10 µg/mL cycloheximide, 1% Na-deoxycholate) were added depending on the experiment, and cells were lysed with the help of a scraper. The lysates were collected and cleared from cellular debris by centrifugation for 5 minutes at 14 000 g at 4° C. The supernatants were transferred into new tubes and the RNA content of each lysate was assessed by measuring the absorbance at 260 nm using Thermo Scientific NanoDrop. Next, since lysates were obtained in hypotonic conditions, the NaCl concentration was adjusted to 100 mM. If not processed immediately, samples were stored at -80 °C.

For RNase I digestion, polysome profiling, and RNA extraction, lysates were treated with RNase I (2/3 Invitrogen AM2295 and 1/3 BioResearch Technologies N6901K) using 7.5 U / a.u. Abs260 of lysate, and incubated for 45 minutes on an orbital shaker at RT. The RNase I activity was inhibited by the addition of 100U of SUPERase·In^{™} RNase Inhibitor (Invitrogen, AM2696). Samples were kept on ice for 10 minutes. In parallel, the sucrose gradient was prepared in cold 13.2 mL polyallomer ultracentrifuge tubes (Beckman) pouring 5.5 mL of 40% w/V sucrose (in 100 mM NaCl, 10 mM MgCl₂, 10 mM Tris/HCl pH 7.5) at the bottom of the tube which was then gently filled with 10% w/V sucrose (in 100 mM NaCl, 10 mM MgCl₂, 10 mM Tris/HCl pH 7.5). The tubes were slowly tilted to a horizontal position and maintained for 2 hours at 4°C to allow the gradient formation. After 2 hours, they were placed in a vertical position and kept on ice. From the top, 700 µL of sucrose were removed and the cytoplasmic lysate was gently loaded drop by drop. The samples were ultracentrifuged for 1 hour and 30 minutes at 40 000 rpm at 4 °C in Beckman Optima XPN-100 Ultracentrifuge in the SW41 rotor. After ultracentrifugation, gradients were fractionated using a Teledyne ISCO model 160 fractionator equipped with a UA-6 UV/ VIS detector to measure absorbance at 254 nm. For each sample, 12 fractions were collected, each of 1000 µL in volume. Fractions corresponding to the 80S monosome were collected, treated with 100 µL/mL of 10% SDS and 5 µL/mL of Proteinase K (PanReac AppliChem, #A4392) and incubated at 37 °C for 1 hour and 30 minutes. Next, 250 µL/mL of Phenol:Chloroform:Isoamyl Alcohol (Sigma, P3803) was added and the samples were centrifuged for 10 min at 10 621 g at 4°C. The upper aqueous phase was transferred into a new tube and RNA was precipitated in 1 mL of isopropanol with the addition of 1 µL of GlycoBlue Coprecipitant (Invitrogen, AM9516), for each sample. Upon precipitation at -80 °C overnight, samples were centrifuged at 15 294 g for 45 minutes at 4°C. The pellets were washed with 500 µL of cold 80% ethanol, and, upon complete drying, RNA was solubilized in 5 µL of nuclease-free water.

For Size selection and PAGE purification of RPFs, RNA from each sample was mixed with the 2x denaturing TBE-Urea Sample Buffer (Invitrogen, LC6876) and denatured at 80 °C for 90 seconds. RNA was run on a 15% TBE-Urea gel (Invitrogen, EC6885BOX) for 1 hour and 10 minutes at 170V. After fast staining with SYBR Gold Nucleic Acid Gel Stain (Invitrogen, S11494), the gel was visualized using Bio-Rad Chemidoc. The fragments between 28-32 nt were excised and the band was dissolved in 400 µL of nuclease-free water, 40 µL of ammonium acetate 5M, and 2 µL of SDS 10% using a pestle. Samples were left overnight at 4 °C in rotation. The next day, they were transferred onto Ultrafree Centrifugal Filters Units 0.22 µm (Millipore, UFC30GV0S) and centrifuged according to the manufacturer's protocol. The RNA was precipitated in 700 µL of isopropanol, as previously described, and finally resuspended in 20 µL of nuclease-free water.

For the library preparation with standard RiboSeq, 40 ng of RPFs underwent the steps of sequencing library preparation according to the Immagina Biotechnology protocol for LaceSeq^{™} (LS001-12). Upon amplification, libraries were purified, as previously described, using 8% TAE gel electrophoresis (40 minutes at 150V) and the dsDNA was solubilized in 12 µL of nuclease-free water. Their quality was assessed using High Sensitivity DNA CHIPS (Agilent Technologies, #5067-4626) on the Agilent 2100 Bioanalyzer. Eventually, libraries were analyzed by single-read sequencing 100 cycles with Illumina NovaSeq 6000 by the Next Generation Sequencing Facility (CIBIO, University of Trento, Italy).

### In vitro RiboWich and in vitro multiplexed RiboWich

Cytoplasmatic lysates from NT and HT HE293T cells, cortical neurons (CNs), and astrocytes were prepared as previously described (step i). RNase I treatment (step ii), nuclease inhibition (step iii), and polysome profiling were performed as previously illustrated. Ribosomes were purified (step iiia) from fractions corresponding to the 80S monosome by ultracentrifuging for 1 hour and 30 minutes at 100 000 rpm at 4 °C in the Benchtop Beckmann Ultracentrifuge, Optima TLX ultracentrifuge using the TLA100.2 rotor. Supernatants were discarded, and ribosomes were resuspended in 10-40 µL of the *in vitro* Ribosome Solution (75 mM KCl, 50 mM Tris-HCl pH 8.1, 5 mM MgCl₂ in DEPC, freshly supplemented with 10 mM DTT (Sigma Aldrich), 10 µg/mL cycloheximide, 0.2 U/µL RiboLock RNase Inhibitor (Thermo Scientific)). Ribosome concentrations were determined by measuring the absorbance at 260 nm with the Thermo Scientific NanoDrop and employing the published ribosome extinction coefficient (5x10⁷ cm⁻¹ M⁻¹) (Algire et al., 2002). Ribosomes were eventually stored at -80°C, after fast freezing in liquid nitrogen.

For the *in vitro* RiboWich experiment in HEK293T, ribosomes were incubated for 2 hours at 37 °C in rotation with the *in vitro* RiboWich Ligation Solution *(in vitro* Ribosome Solution supplemented with the sequencing adaptor (LaceSeq^{™}, LS001-12) in a ratio 1:2 (adaptor: ribosomes), 0.15 mM GTP (Invitrogen, AM8130G), 1.8 mM MnCl₂, and 1 µL of Rtcb enzyme (New England Biolabs, M0458S)) (step iv). For the *in vitro* multiplexed RiboWich experiment in CNs and astrocytes, ribosomes were incubated for 2 hours at 37°C in rotation with the *in vitro* RiboWich Solution with multiplexed sequencing adaptors (Immagina Biotechnology Multiplexed LaceSeq^{™}, MX001-36) in a ratio 1:4 (adaptor: ribosomes), and the rest of the reagents as described above (step iv).

Upon incubation, RNA was extracted either with the TRIzol reagent (Thermo Fisher Scientific, 15596026) following the manufacturer's protocol (gel approach) or with RNA Clean & Concentrator^{™}-5 (Zymo Research, R1013, R1014, R1015, or R1016) following manufacturer's instructions for small RNA purification (gel-free approach), respectively for HE293T, and CNs and astrocytes. For the HEK293T experiment (gel approach), upon extraction, samples were resuspended into 5 µL of nuclease-free water and run on a 15% TBE-Urea gel (Invitrogen, EC6885BOX) and purified as previously described. The RNA was then resuspended in 20 µL of nuclease-free water. For the CNs and astrocytes experiment (gel-free approach), RNA was extracted, purified via columns, and finally eluted in 20 µL of nuclease-free water. In both abovementioned cases, the library preparation continued with the steps of 5' phosphorylation, circularization, reverse transcription, and PCR amplification (according to the Immagina Biotechnology protocol for LaceSeq^{™} (LS001-12)). Libraries were finally purified, quantified, and sent for sequencing as previously described (Immagina Biotechnology protocol for LaceSeq^{™} (LS001-12)).

### In-lysate multiplexed RiboWich

HEK293T cells for NT and HT conditions were treated as previously described. Upon incubation, the medium was removed, and cells were washed once with the Preserving Solution (PBS supplemented with 10 µg/mL of cycloheximide). Then, trypsin-EDTA in PBS was added, and cells were incubated for 5-10 minutes at 37°C. Cells were resuspended in the medium supplemented with 10 µg/mL of cycloheximide. Cells were counted and centrifuged at 950 rpm for 5 minutes at RT in ALC Refrigerated Centrifuge PK 120R using swinging bucket rotor E-024 (0.161m radius). Cells were resuspended in the Preserving Solution (PBS supplemented with 10 µg/mL cycloheximide), and 100 000 cells were collected and lysed with 50 µL of RiboWich Cytoplasmatic Lysis Buffer (step i) (10 mM Tris-HCl pH 8.1, 5 mM MgCl₂, 10 mM NaCl, 1% Triton X-100, 5 U/mL DNase I (Thermo Scientific), 0.2 U/µL RiboLock RNase Inhibitor (Thermo Scientific), 1 mM DTT (Sigma Aldrich), 10 µg/mL cycloheximide, 1% Na-deoxycholate), for each sample. Lysates were then cleared from cellular debris by centrifugation. Next, the NaCl concentration was adjusted to 75 mM. If not processed immediately, samples were stored at -80 °C. RNase I digestion (step ii) was performed using 0.2 U/µL of RNase I (2/3 Invitrogen AM2295 and 1/3 BioResearch Technologies N6901K), and samples were incubated for 45 minutes on Mixer HC (Starlab) at 600 rpm at RT. The RNase I activity was inhibited (step iii) by the addition of 1 U/µL of SUPERase ·In^{™} RNase Inhibitor (Invitrogen, AM2696). Samples were kept on ice for 10 minutes. Next, the lysates were adjusted to the in-lysate RiboWich Ligation Solution (step iv): Tris-HCl pH 8.1 and DTT concentrations were adjusted to 50 mM and 10 mM, respectively, 0.15 mM GTP (Invitrogen, AM8130G), 1.8 mM MnCl₂, 0.015 µM multiplexed sequencing adaptors were added together with 1 µL of Rtcb enzyme (New England Biolabs, M0458S). The samples were incubated for 2 hours on Mixer HC (Starlab) at 37°C at 600 rpm. Upon the ligation, the samples were pooled, and RNA was directly extracted (with the TRIzol reagent following the manufacturer's protocol, without the need for ribosome purification steps.

The library preparation continued with the phosphorylation at the 5' end, circularization, reverse transcription, and PCR amplification according to the Immagina Biotechnology's protocol for LACE-seq^{™} (LS001-12). Libraries were finally quantified and sent for sequencing as previously described.

### In plate in-lysate multiplexed RiboWich

Cells were seeded in 96-well plates and grown until they reached 40-60% confluence. HEK293T cells for NT and HT conditions were treated as previously described. The treatment with cycloheximide was performed using 100 µg/mL. Upon incubation, the medium was removed from the wells, and cells were washed twice with the Preserving Solution (PBS supplemented with 100 µg/mL cycloheximide). Next, 25 µL of RiboWich Cytoplasmatic Lysis Buffer (step i and ii) (10 mM Tris-HCl pH 8.1, 5 mM MgCl₂, 10 mM NaCl, 1% Triton X-100, 5 U/mL DNase I (Thermo Scientific), 0.4 U/µL RiboLock RNase Inhibitor (Thermo Scientific), 1 mM DTT (Sigma Aldrich), 100 µg/mL cycloheximide, 1% Na-deoxycholate) supplemented with 0.2 U/µL of RNase I (Invitrogen, AM2295) were added onto each well. The plate was incubated for 45 minutes on Mixer HC (Starlab) at 400 rpm at RT. The RNase I activity was inhibited (step iii) by the addition of 1 U/µL of SUPERase ·In^{™} RNase Inhibitor (Invitrogen, AM2696). The plate was incubated for 10 minutes on Mixer HC (Starlab) at 400 rpm at 37°C.

Next, the lysates were adjusted to the in-lysate RiboWich Ligation Solution (step iv): Tris-HCl pH 8.1 and DTT concentrations were adjusted to 50 mM and 10 mM, respectively, 0.15 mM GTP (Invitrogen, AM8130G), 1.8 mM MnCl₂, 0.015 µM multiplexed sequencing adaptors were added together with 0.5 µL of Rtcb enzyme (New England Biolabs, M0458S). The plate was incubated for 2 hours on Mixer HC (Starlab) at 37°C at 400 rpm. Upon the ligation, all the wells were pooled, and RNA was directly extracted using the RNA Clean & Concentrator^{™}-5 kit (Zymo Research, R1013, R1014, R1015, or R1016) following the protocol for Small RNAs and the manufacturer's instructions.

The library preparation continued with the phosphorylation at the 5' end, circularization, reverse transcription, and PCR amplification according to the Immagina Biotechnology's protocol for LACE-seq^{™} (LS001-12). Libraries were finally quantified and sent for sequencing as previously described.

### NGS data analysis

Ribosome profiling analyses were performed using riboWaltz (Lauria et al., 2018).

For RiboSeq and RiboWich, adaptors were removed with CutAdapt v3.7, and UMIs, composed of the first 4 and last 4 nucleotides of the reads, were extracted using UMI-Tools v1.1.2 from the remaining sequences. For multiplexed RiboWich, adaptor sequences were first removed using CutAdapt v3.7. Next, UMIs (comprising the first 4 and last 4 nucleotides of the reads) and barcodes (spanning nucleotides 5 to 12) were extracted from the remaining sequences using UMI-Tools v1.1.2. For all, the first nucleotide was trimmed, and reads shorter than 15 nucleotides were discarded. Reads mapping on rRNA and tRNA sequences were removed, and the remaining reads were mapped on the reference genome using STAR v2.7.10a. The Spearman correlation was computed to compare the ribosome coverage of the transcripts across different samples. Annotation enrichment analysis with Gene Ontology terms was performed with the EnrichR platform.

### Annotation of upstream ORFs

Active non-canonical upstream Open Reading Frames (uORFs) were defined as sequences in the transcriptome with a start codon (either AUG or one of the 4 most common alternatives: CUG, UUG, GUG, ACG) detected before the canonical one, a stop codon in frame with respect to the start site, and with a length multiple of 3 nucleotides.

### Identification of active upstream ORFs

Active uORFs in RiboSeq and RiboWich were identified using a four-step filtering process, starting with all uORFs with at least one mapped read. uORFs meeting the following criteria were retained: (i) RPKM > 10; (ii) at least 25% of total reads mapping to the start site in the HT condition; (iii) over 50% of total reads in the correct frame in the NT condition; and (iv) criteria (i)-(iii) satisfied in both RiboSeq and RiboWich datasets. The identified ORFs were crossreferenced with the RiboUORF database. The RibouORF database (Liu, Qi, et al., 2023), obtained from https://rnainformatics.org.cn/RiboUORF. Additionally, the list of 274 uORFs associated with at least one peptide identified in proteomic datasets was sourced from Mudge et al., 2022.

In the following two detailed protocols for performing RiboWich are provided.

### In-lysate RiboWich: Detailed protocol

For in-lysate RiboWich, we recommend using:
- **Non-multiplexing approach:** ≥ 500,000 cells per sample.
- **Multiplexing approach:** 1 ≤ number of cells ≤ 500,000 per sample.

### Lysate collection, RNase treatment, and RNase inhibition - Steps (i), (ii), and (iii)

1.Culture your cells of interest in appropriate flasks, plates, or dishes until they reach 40-80% confluence.
2.Treat the cells with 10-200 µg/mL cycloheximide (in nuclease-free water) and incubate at 37 °C for 2-5 minutes.
3.Remove the medium and wash with the **Preserving Solution** (Table 3) at least once.

**Table 3**

| **Buffer components** | **Final concentrations** |
|---|---|
| Cycloheximide* | 10-200 µg/mL |
| In Phosphate Buffered Saline (PBS) | |

| | |
|---|---|
| **This component is prepared in nuclease-free water.* | |

If in flask or dish:
4.Incubate the cells with 0.25% trypsin-EDTA for 5-10 minutes at 37 °C.
5.Add medium supplemented with 10-200 µg/mL cycloheximide and transfer the cell suspension into a Falcon tube.
6.Centrifuge the cells at 161 g for 5 minutes at RT.
7.Discard the supernatant and resuspend the cell pellet in the Preserving Solution.
8.*Optional:* Add 1% BSA (in nuclease-free water) to favour the pelleting.
9.Centrifuge again.
10.Remove the supernatant and add 10-500 µL (depending on the cell number and cell type) of **RiboWich Cytoplasmatic Lysis Buffer** (Table 4) directly onto the cell pellet. Mix well by pipetting up and down to favour the lysis.

**Table 4**

| **Buffer components** | **Final concentrations** | **Preferred concentrations** |
|---|---|---|
| TRIS HCl (pH 8.1)* | 5-15 mM | 10 mM (pH 7.5) |
| MgCl₂* | 1-5 mM | 5 mM |
| NaCl* | 5-15 mM | 10 mM |
| Triton X-100* | 0.75-1.25 % | 1 % |
| DTT* | 0.75-1.25 mM | 1 mM |
| Cycloheximide* | 10-200 µg/mL | 10 µg/mL |
| Dnase I | 1-10 U/mL | 5 U/mL |
| RiboLock RNase Inhibitor | 0.1-0.5 U/µL | 0.2 U/µL |
| Na deoxycholate* | 0.75-1.25 % | 1 % |
| In nuclease-free water | | |

| | | |
|---|---|---|
| **This component is prepared in nuclease-free water.* | | |

11. Incubate the lysates on ice for at least 10 minutes.
12. Centrifuge at 10,000 - 14,000 g for 5-10 minutes and collect the supernatants.
13. Measure the Abs 260 nm of the lysate to estimate the amount of RNA.
14. Safe stopping point. Store the lysates at -80 °C. As an alternative, proceed with the following steps.
15.Treat the lysates with 0.1-0.4 U RNase I/µL, MNase, or any other endonuclease that hydrolyzes single-stranded RNA to nucleoside 3'-monophosphate or cyclic phosphate.
16.Incubate the samples either at RT for 30-60 minutes or at 37 °C for 5-20 minutes in a thermoshaker with shaking at 300-600 rpm. Continue with step 17.

If in plate:
4. Remove the residual Preserving Solution from the wells and add 5-100 µL (depending on the plate, cell number, and cell type) of **RiboWich Cytoplasmatic Lysis Buffer** (Table 4) supplemented with 0.1-0.4 U RNase I/µL, MNase, or any other endonuclease that hydrolyzes single-stranded RNA to nucleoside 3'-monophosphate or cyclic phosphate onto each well.
5. Incubate the plate either at RT for 30-60 minutes or at 37°C for 5-20 minutes in a thermoshaker with shaking at 300-600 rpm. Continue with step 17.

17.Inhibit the activity of the RNase I by adding 0.1-2 U/µL SUPERase ·In^{™} RNase Inhibitor (Invitrogen) and incubate the samples either at 37 °C for 5-20 minutes in a thermoshaker with shaking at 300-600 rpm or on ice for at least 10 minutes. As an alternative, freeze them for at least 30 minutes at -80 °C or use any other method that ensures efficient endonuclease inhibition while preserving ribosome integrity.

### Adaptor ligation and RNA extraction - Step (iv)

18.Adjust the RiboWich Cytoplasmatic Lysis Buffer for the ligation reaction as follows and incubate the lysates in the **in-lysate RiboWich Ligation Solution** (Table 5) for at least 1 hour at 37 °C in a thermoshaker with shaking at 300-600 rpm.

**Table 5**

| **Buffer components** | **Final concentrations** | **Preferred concentrations** |
|---|---|---|
| TRIS HCl (pH 8.1)* | 5-100 mM | 50 mM (pH 7.5) |
| DTT* | 5-20 mM | 10 mM |
| GTP** | 0.05-0.4 mM | 0.15 mM |
| MnCl₂* | 0.5-4 mM | 1.8 mM |
| Adaptor / multiplexed adaptor*^{/}^ | 0.01-0.04 µM*** | 0.015 µM |
| RtcB ligase** | 0.5-2 µL/50 µL | 1 µL/50 µL |

| | | |
|---|---|---|
| **This component is prepared in nuclease-free water.* ***Those reagents come from the RtcB Ligase kit - New England Biolabs (M04585).* ****This concentration is calculated for each sample and depends on the starting material.* *^ For the sequences, refer to the tables 26 and 27 "adaptors and primers used for library preparation".* | | |

19. In the case of the multiplexing in-lysate RiboWich, pool together the lysates from ligation reactions with differently barcoded adaptors.

20. Extract the RNA and purify the reaction through the RNA Clean & Concentrator^{™}-5 kit (Zymo Research, R1013, R1014, R1015, or R1016) following the protocol for Small RNAs and the manufacturer's instructions. Finally, elute in 6-21 µL of nuclease-free water.

As an alternative to the above-mentioned column-based method for RNA extraction, you could follow one of the alternatives to enrich for the fragments of interest:
A. Perform polysome profiling, according to (Lauria et al., 2020). Successively, extract the RNA from the sucrose fraction(s) corresponding to 80S monosomes with acid phenol: chloroform followed by isopropanol precipitation, according to (Clamer et al., 2018).
B. Perform active ribosome isolation with the RiboLace kit (Immagina BioTechnology, # RL00P-12 or # RL00S-04) following the manufacturer's instructions.
C. Any other method can be employed to purify the ligated REF within the ribosome.

### Optional: Gel-based size selection and purification

21. The extracted RNA could be run on a 15% TBE-urea polyacrylamide gel.

22. Pre-run the gel at 200 V for 30 minutes in TBE prepared with nuclease-free water.

23. Mix the samples with the 2X denaturing TBE-Urea Sample Buffer and denature them at 80 °C for 90 seconds.

24. Load the samples and the 20-100 ladder and run the gel at 170 V for 70 minutes.

25. Stain the gel for 5 minutes with SYBR Gold in TBE and visualize the RNA using a UV transilluminator.

26. Excise ligated fragments (~ 80 nt for non-multiplexing and ~ 90 nt for multiplexing approaches) from gel and dissolve the gel band in 400 µL nuclease-free water, 40 µL ammonium acetate 5 M (in nuclease-free water), and 2 µL SDS 10 % (in nuclease-free water) using a pestle.

27. Leave them overnight at 4 °C in rotation.

28. The following day, transfer the samples into Ultrafree-MC Centrifugal Filter Units and centrifuge them for 3 minutes at 2300 g to separate any gel residue from the RNA.

29. Add 700 µL of isopropanol and 2 µL of Glyco-blue.

30. Put the samples at -80 °C for at least 1 hour.

31. Centrifuge at 10,000-14,000 g for 45 minutes at 4 °C.

32. Wash the pellet with 500 µL of 80% ethanol (in nuclease-free water).

33. Centrifuge at 10,000-14,000 g for 5 minutes at 4 °C.

34. Remove the supernatant and let the pellet air dry for at least 15 minutes.

35. Solubilize the pellets in 20 µL of nuclease-free water.

### Library preparation (adapted from LaceSeq, Immagina BioTechnology, LS001-12)

### 5' phosphorylation

36. Prepare the phosphorylation reaction (final volume = 50 µL) on ice as follows (Table 6):

**Table 6**

| **Reaction components** | **Volumes to add** |
|---|---|
| Ligated RNA | 20 µL |
| T4 Polynucleotide Kinase Reaction Buffer* | 5 µL (from the 10X stock) |
| ATP | 5 µL (from the 10 mM stock) |
| PNK 3' minus enzyme* | 1 µL |
| Nuclease-free water | Up to 50 µL |

| | |
|---|---|
| **Those reagents come from the T4 Polynucleotide Kinase (3' phosphatase minus) kit - New England Biolabs (M0236S or M0236L).* | |

37. Incubate the reaction for 1 hour at 37 °C.

38. Purify the reaction through the RNA Clean & Concentrator^{™}-5 kit (Zymo Research, R1013, R1014, R1015, or R1016) following the protocol for Small RNAs and the manufacturer's instructions. Finally, elute in 10 µL of nuclease-free water.

### Circularization

39. Prepare the circularization reaction (final volume = 21 µL) on ice as follows (Table 7):

**Table 7**

| **Reaction components** | **Volumes to add** |
|---|---|
| Phospho RNA | 9 µL |
| BLB buffer* | 2 µL (from the 10X stock) |
| ATP | 1 µL (from the 1 mM stock) |
| PEG8000** | 8 µL (from the 50 % stock) |
| T4 RNA ligase 1 enzyme** | 1 µL |

| | |
|---|---|
| **BLB buffer 10X: 500 mM TRIS HCl pH 8.1, 10 mM MgCl₂, 10 mM DTT in nuclease-free water.* ***Those reagents come from the T4 RNA Ligase 1 (ssRNA Ligase) kit* - *New England Biolabs (M0204S or M0204L).* | |

40. Incubate the reaction for 2 hours at 25 °C.

41. Add 30 µL of nuclease-free water.

42. Purify the reaction through the RNA Clean & Concentrator^{™}-5 kit (Zymo Research, R1013, R1014, R1015, or R1016) following the protocol for Small RNAs and the manufacturer's instructions. Finally, elute in 13 µL of nuclease-free water.

43. Safe stopping point. Store the circ RNA at -80 °C. As an alternative, proceed immediately with the following reaction.

### Reverse transcription

44. Prepare the reverse transcription reaction (final volume = 14 µL) on ice as follows (Table 8):

**Table 8**

| **Reaction components** | **Volumes to add** |
|---|---|
| Circ RNA | 12 µL |
| dNTPs | 1 µL (from the 10 mM stock) |
| RT primer" | 1 µL (from the 10 µM stock) |

| | |
|---|---|
| *^ For the sequences, refer to the tables 26 and 27 "adaptors and primers used for library preparation".* | |

45. Incubate the pre-mix at 70 °C for 5 minutes and then transfer it on ice for at least 1 minute.

46. Supplement the reverse transcription reaction (final volume = 20 µL) on ice as follows (Table 9):

**Table 9**

| **Reaction components** | **Volumes to add** |
|---|---|
| First-Strand Buffer* | 4 µL (from the 5X stock) |
| DTT* | 1 µL (from the 0.1 M stock) |
| Super Script III enzyme* | 1 µL |

| | |
|---|---|
| **Those reagents come from the SuperScript^{™} III Reverse Transcriptase kit - Invitrogen (18080093, 18080044 or 18080085).* | |

47. Incubate for 40 minutes at 50 °C, then heat-inactivate for 10 minutes at 80 °C (10 minutes at 70 °C for gel-free approaches).

### Optional: Exonuclease I digestion (mandatory only for gel-free approaches)

48. Transfer on ice for at least 1 minute.

49. Add 0.5 µL of Exo I enzyme / 20 µL reaction.

50. Incubate the solution at 37 °C for 1 hour and then at 80 °C for 20 minutes.

51. Safe stopping point. Store the cDNA at 4 °C overnight or -20 °C for up to one week. As an alternative, proceed immediately with the following reaction.

52. In the case of the multiplexing in-lysate RiboWich, pool together the reverse transcription reactions with differently barcoded RT primers.

53. Purify cDNA either by precipitation or using column-based approaches.

### PCR1 amplification

54. Prepare the PCR1 reaction (final volume = 100 µL) on ice as follows (Table 10):

**Table 10**

| **Reaction components** | **Volumes to add** |
|---|---|
| cDNA | 20 µL |
| Phusion^{™} High-Fidelity PCR Master Mix with HF Buffer* | 50 µL (from the 2X stock) |
| F1 primer" | 0.8 µL (from the 10 µM stock) |
| R1 primer" | 0.8 µL (from the 10 µM stock) |
| Nuclease-free water | Up to 100 µL |

| | |
|---|---|
| **Those reagents come from the Phusion^{™} High-Fidelity PCR Master Mix with HF Buffer kit - New England Biolabs (M0531S or M0531L).* *^ For the sequences, refer to the tables 26 and 27 "adaptors and primers used for library preparation".* | |

55. Place the tube in a thermal cycler and run the following program (Table 11):

**Table 11**

| **Step** | **Temp** | **Time** |
|---|---|---|
| Initial denaturation | 98 °C | 1 min |
| 6-12 cycles* | 98 °C | 30 sec |
| | 61 °C | 30 sec |
| | 72 °C | 10 sec |
| Hold | 4 °C | ∞ |

| | | |
|---|---|---|
| **The number of cycles depends on the starting material.* | | |

56. Purify the PCR reaction by using 1.6X Agencourt AMPure XP beads (Beckman Coulter, A63880, A63881, or A63882) with respect to PCR1 reaction's volume, following the manufacturer's instructions. Finally, elute in 50 µL of nuclease-free water.

### PCR2 amplification

57. Prepare the PCR2 reaction on ice as follows (Table 12):

**Table 12**

| **Reaction components** | **Volumes to add** |
|---|---|
| dsDNA | 49 µL |
| Phusion^{™} High-Fidelity PCR Master Mix with HF Buffer* | 50 µL |
| UDI | 1 µL |

| | |
|---|---|
| **Those reagents come from the Phusion^{™} High-Fidelity PCR Master Mix with HF Buffer kit - New England Biolabs (M0531S or M0531L).* | |

58. Place the tube in a thermal cycler and run the following program (Table 13):

**Table 13**

| **Step** | **Temp** | **Time** |
|---|---|---|
| Initial denaturation | 98 °C | 1 min |
| 5-7 cycles* | 98 °C | 30 sec |
| | 60 °C | 30 sec |
| | 72 °C | 10 sec |
| Hold | 4 °C | ∞ |

| | | |
|---|---|---|
| **The number of cycles depends on the starting material.* | | |

59. Purify the PCR reaction by using NucleoSpin Gel and PCR Clean-Up (MACHEREY-NAGEL, 740609.50) kit following the manufacturer's instructions. Finally, elute in 16 µL of nuclease-free water.

### Optional: Gel-based library purification

60. The dsDNA library could be run on an 8% TAE polyacrylamide gel.

61. Mix the samples with the 6x DNA Loading Dye.

62. Load the samples and the Ultra Low Range DNA Ladder and run the gel at 150 V for 45 minutes.

63. Stain the gel for 5 minutes with SYBR Gold in TAE and visualize the RNA using a UV transilluminator.

64. Excise the library at ~ 200 bp (or ~ 210 bp in the case of the multiplexing protocol) from the gel and dissolve the gel band in 400 µL nuclease-free water, 40 µL ammonium acetate 5 M (in nuclease-free water), and 2 µL SDS 10 % (in nuclease-free water) using a pestle.

65. Leave them overnight at 4 °C in rotation.

66. The following day, transfer the samples into Ultrafree-MC Centrifugal Filter Units and centrifuge them for 3 minutes at 2300 g to separate any gel residue from the DNA.

67. Add 700 µL of isopropanol and 2 µL of Glyco-blue.

68. Put the samples at -80 °C for at least 1 hour.

69. Centrifuge at 10,000-14,000 g for 45 minutes at 4 °C.

70. Wash the pellet with 500 µL of 80% ethanol (in nuclease-free water).

71. Centrifuge at 10,000-14,000 g for 5 minutes at 4 °C.

72. Remove the supernatant and let the pellet air dry for at least 15 minutes.

73. Solubilize the pellets in 15 µL of nuclease-free water.

### Library quality control, quantification, and sequencing

74. Run the samples on the Agilent 2100 Bioanalyzer using High Sensitivity DNA CHIPS (Agilent Technologies #5067-4626) following the manufacturer's instructions.

75. Eventually, analyze the libraries by single-read sequencing 100 cycles with Illumina NovaSeq 6000 (or any other Illumina platform) by the Next Generation Sequencing.

### In vitro RiboWich: Detailed protocol

For *in vitro* RiboWich, we recommend using:
- **Non-multiplexing approach:** ≥ 500,000 cells per sample.
- **Multiplexing approach:** 100,000 ≤ number of cells ≤ 500,000 per sample.

### Lysate collection, RNase treatment, and RNase inhibition -Steps (i), (ii), and (iii)

1. Culture your cells of interest in appropriate plates or dishes until they reach 40-80% confluence.
2. Treat the cells with 10-200 µg/mL cycloheximide (in nuclease-free water) and incubate at 37 °C for 2-5 minutes.
3. On ice, remove the medium and wash with the **Preserving Solution** (table 14) at least once.

**Table 14**

| **Buffer components** | **Final concentrations** |
|---|---|
| Cycloheximide* | 10-200 µg/mL |
| In Phosphate Buffered Saline (PBS) | |

| | |
|---|---|
| **This component is prepared in nuclease-free water.* | |

4. Add 10-500 µL (depending on the cell number and cell type) of **RiboWich Cytoplasmatic Lysis Buffer** (Table 15), lyse the cells using a scraper, and collect the lysates into new tubes.

**Table 15**

| **Buffer components** | **Final concentrations** | **Preferred concentrations** |
|---|---|---|
| TRIS HCl (pH 8.1)* | 5-15 mM | 10 mM (pH 7.5) |
| MgCl₂* | 5-15 mM | 10 mM |
| NaCl* | 5-15 mM | 10 mM |
| Triton X-100* | 0.75-1.25 % | 1 % |
| DTT* | 0.75-1.25 mM | 1 mM |
| Cycloheximide* | 10-200 µg/mL | 10 µg/mL |
| Dnase I | 1-10 U | 5 U/mL |
| RiboLock RNase Inhibitor | 0.1-0.5 U/µL | 0.2 U/µL |
| Na deoxycholate* | 0.75-1.25 % | 1 % |
| In nuclease-free water | | |

| | | |
|---|---|---|
| **This component is prepared in nuclease-free water.* | | |

5. Centrifuge at 10,000 - 14,000 g for 5-10 minutes and collect the lysates.
6. Measure the Abs 260 nm of the lysate to estimate the amount of RNA.
*7. Optional:* Adjust the NaCl concentration from 5-15 mM to 75-100 mM.
8. Safe stopping point. Store the lysates at -80 °C. As an alternative, proceed with the following steps.
9. Treat the lysates with 1-10 U RNase I / Abs (a.u.), MNase, or any other endonuclease that hydrolyzes single-stranded RNA to nucleoside 3'-monophosphate or cyclic phosphate.
10. Incubate the samples either at RT for 30-60 minutes or at 37 °C for 5-20 minutes in a thermoshaker with shaking at 300-600 rpm.
11. Inhibit the activity of the RNase I by adding 0.1-2 U/µL SUPERase·In^{™} RNase Inhibitor (Invitrogen) and incubating them either at 37 °C for 5-20 minutes in a thermoshaker with shaking at 300-600 rpm or on ice for at least 10 minutes. As an alternative, freeze the samples for at least 30 minutes at -80 °C or use any other method that ensures efficient endonuclease inhibition while preserving ribosome integrity.

### Ribosome isolation and purification (step iiia)

12. Perform polysome profiling and collect the 80S fraction (s) (Lauria et al., 2020). Next, pellet the ribosomes with a second round of ultracentrifugation at 435,680 g for 1 hour and 30 minutes at 4 °C. As an alternative, exploit any other method for ribosome isolation.

13. Resuspend the ribosomes in 10-50 µL (depending on the expected amount of ribosomes) of ***in vitro* Ribosome Solution** (Table 16).

**Table 16**

| **Buffer components** | **Final concentrations** | **Preferred concentrations** |
|---|---|---|
| TRIS HCl (pH 8.1) ***** | 5-100 mM | 50 mM (pH 8.1) |
| MgCl₂* | 1-5 mM | 5 mM |
| KCl* | 5-100 mM | 75 mM |
| DTT* | 1-20 mM | 10 mM |
| Cycloheximide* | 10-200 µg/mL | 10 µg/mL |
| RiboLock | 0.1-0.5 U/µL | 0.2 U/µL |
| In nuclease-free water | | |

| | | |
|---|---|---|
| **This component is prepared in nuclease-free water.* | | |

14. Measure the Abs 260 nm to estimate the amount of ribosomes.

15. Safe stopping point. Fast-freeze the ribosomes in liquid nitrogen and store them at -80 °C. As an alternative, proceed with the following steps.

### Adaptor ligation and RNA extraction - Step (iv)

16. Adjust the *in vitro* Ribosome Solution for the ligation reaction on ice as follows and incubate the ribosomes in the ***in vitro* RiboWich Ligation Solution** (Table 17) for at least 1 hour at 37 °C in a thermoshaker with shaking at 300-600 rpm.

**Table 17**

| **Buffer components** | **Final concentrations** | **Preferred concentrations** |
|---|---|---|
| GTP** | 0.05-0.4 mM | 0.15 mM |
| MnCl₂* | 0.5-4 mM | 1.8 mM |
| Adaptor / multiplexed adaptor*^{/}^ | 0.1 nM - 0.01 µM*** | |
| RtcB ligase** | 0.5-2 µL/50 µL | 1 µL/50 µL |

| | | |
|---|---|---|
| **This component is prepared in nuclease-free water.* ***Those reagents come from the RtcB Ligase kit - New England Biolabs (M04585).* ****This concentration is calculated for each sample and depends on the starting material - non-multiplexed sequencing adaptor (LaceSeq^{™}, LS001-12) in a ratio 1:2, or multiplexed sequencing adaptor (Multiplexed LaceSeq^{™}, MX001-36) in a ratio 1:4 (adaptor: ribosomes).* *^ For the sequences, refer to the tables 26 and 27 "adaptors and primers used for library preparation".* | | |

17. In the case of the multiplexing *in vitro* RiboWich, pool together the ribosomes from ligation reactions with differently barcoded adaptors.

18. Extract the RNA and purify the reaction through the RNA Clean & Concentrator^{™}-5 kit (Zymo Research, R1013, R1014, R1015, or R1016) following the protocol for Small RNAs and the manufacturer's instructions. Finally, elute in 21 µL of nuclease-free water. As an alternative, you can use any other method of RNA isolation.

### Optional: Gel-based size selection and purification

19. The extracted RNA could be run on a 15% TBE-urea polyacrylamide gel.

20. Pre-run the gel at 200 V for 30 minutes in TBE prepared with nuclease-free water.

21. Mix the samples with the 2X denaturing TBE-Urea Sample Buffer and denature them at 80 °C for 90 seconds.

22. Load the samples and the 20-100 ladder and run the gel at 170 V for 70 minutes.

23. Stain the gel for 5 minutes with SYBR Gold in TBE and visualize the RNA using a UV transilluminator.

24. Excise ligated fragments (~ 80 nt for non-multiplexing and ~ 90 nt for multiplexing approaches) from gel and dissolve the gel band in 400 µL nuclease-free water, 40 µL ammonium acetate 5 M (in nuclease-free water), and 2 µL SDS 10 % (in nuclease-free water) using a pestle.

25. Leave them overnight at 4 °C in rotation.

26. The following day, transfer the samples into Ultrafree-MC Centrifugal Filter Units and centrifuge them for 3 minutes at 2300 g to separate any gel residue from the RNA.

27. Add 700 µL of isopropanol and 2 µL of Glyco-blue.

28. Put the samples at -80 °C for at least 1 hour.

29. Centrifuge at 10,000-14,000 g for 45 minutes at 4 °C.

30. Wash the pellet with 500 µL of 80% ethanol (in nuclease-free water).

31. Centrifuge at 10,000-14,000 g for 5 minutes at 4 °C.

32. Remove the supernatant and let the pellet air dry for at least 15 minutes.

33. Solubilize the pellets in 20 µL of nuclease-free water.

### Library preparation (adapted from LaceSeq, Immagina BioTechnology, LS001-12)

### 5' phosphorylation

34. Prepare the phosphorylation reaction (final volume = 50 µL) on ice as follows (Table 18):

**Table 18**

| **Reaction components** | **Volumes to add** |
|---|---|
| Ligated RNA | 20 µL |
| T4 Polynucleotide Kinase Reaction Buffer** | 5 µL (from the 10X stock) |
| ATP | 5 µL (from the 10 mM stock) |
| PNK 3' minus enzyme* | 1 µL |
| Nuclease-free water | Up to 50 µL |

| | |
|---|---|
| **Those reagents come from the T4 Polynucleotide Kinase (3' phosphatase minus) kit - New England Biolabs (M0236S or M0236L).* | |

35. Incubate the reaction for 1 hour at 37 °C.

36. Purify the reaction through the RNA Clean & Concentrator^{™}-5 kit (Zymo Research, R1013, R1014, R1015, or R1016) following the protocol for Small RNAs and the manufacturer's instructions. Finally, elute in 10 µL of nuclease-free water.

### Circularization

37. Prepare the circularization reaction (final volume = 21 µL) on ice as follows (Table 19):

**Table 19**

| **Reaction components** | **Volumes to add** |
|---|---|
| Phospho RNA | 9 µL |
| BLB buffer* | 2 µL (from the 10X stock) |
| ATP | 1 µL (from the 1 mM stock) |
| PEG8000** | 8 µL (from the 50 % stock) |
| T4 RNA ligase 1 enzyme** | 1 µL |

| | |
|---|---|
| **BLB buffer 10X: 500 mM TRIS HCl pH 8.1, 10 mM MgCl₂, 10 mM DTT in nuclease-free water.* ***Those reagents come from the T4 RNA Ligase 1 (ssRNA Ligase) kit - New England Biolabs (M0204S or M0204L).* | |

38. Incubate the reaction for 2 hours at 25 °C.

39. Add 30 µL of nuclease-free water.

40. Purify the reaction through the RNA Clean & Concentrator^{™}-5 kit (Zymo Research, R1013, R1014, R1015, or R1016) following the protocol for Small RNAs and the manufacturer's instructions. Finally, elute in 13 µL of nuclease-free water.

41. Safe stopping point. Store the circ RNA at -80 °C. As an alternative, proceed immediately with the following reaction.

### Reverse transcription

42. Prepare the reverse transcription reaction (final volume = 14 µL) on ice as follows (Table 20):

**Table 20**

| **Reaction components** | **Volumes to add** |
|---|---|
| Circ RNA | 12 µL |
| dNTPs | 1 µL (from the 10 mM stock) |
| RT primer" | 1 µL (from the 10 µM stock) |

| | |
|---|---|
| *^ For the sequences, refer to the tables 26 and 27 "adaptors and primers used for library preparation".* | |

43. Incubate the pre-mix at 70 °C for 5 minutes and then transfer it on ice for at least 1 minute.

44. Supplement the reverse transcription reaction (final volume = 20 µL) on ice as follows (Table 21) :

**Table 21**

| **Reaction components** | **Volumes to add** |
|---|---|
| First-Strand Buffer* | 4 µL (from the 5X stock) |
| DTT* | 1 µL (from the 0.1 M stock) |
| Super Script III enzyme* | 1 µL |

| | |
|---|---|
| **Those reagents come from the SuperScript^{™} III Reverse Transcriptase kit - Invitrogen (18080093, 18080044, or 18080085).* | |

45. Incubate for 40 minutes at 50 °C, then heat-inactivate for 10 minutes at 80 °C (10 minutes at 70 °C for gel-free approaches).

### Optional: Exonuclease I digestion (mandatory only for gel-free approaches)

46. Transfer on ice for at least 1 minute.

47. Add 0.5 µL of Exo I enzyme / 20 µL reaction.

48. Incubate the solution at 37 °C for 1 hour, and then at 80 °C for 20 minutes.

49. Safe stopping point. Store the cDNA at 4 °C overnight or -20 °C for up to one week. As an alternative, proceed immediately with the following reaction.

### PCR1 amplification

50. Prepare the PCR1 reaction (final volume = 100 µL) on ice as follows (Table 22):

**Table 22**

| **Reaction components** | **Volumes to add** |
|---|---|
| cDNA | 20 µL |
| Phusion^{™} High-Fidelity PCR Master Mix with HF Buffer* | 50 µL (from the 2X stock) |
| F1 primer" | 0.8 µL (from the 10 µM stock) |
| R1 primer" | 0.8 µL (from the 10 µM stock) |
| Nuclease-free water | Up to 100 µL |

| | |
|---|---|
| **Those reagents come from the Phusion^{™} High-Fidelity PCR Master Mix with HF Buffer kit - New England Biolabs (M0531S or M0531L).* *^ For the sequences, refer to the tables 26 and 27 "adaptors and primers used for library preparation".* | |

51. Place the tube in a thermal cycler and run the following program (Table 23):

**Table 23**

| **Step** | **Temp** | **Time** |
|---|---|---|
| Initial denaturation | 98 °C | 1 min |
| 6-12 cycles* | 98 °C | 30 sec |
| | 61 °C | 30 sec |
| | 72 °C | 10 sec |
| Hold | 4 °C | ∞ |

| | | |
|---|---|---|
| **The number of cycles depends on the starting material.* | | |

52. Purify the PCR reaction by using 1.6X Agencourt AMPure XP beads (Beckman Coulter, A63880, A63881, or A63882) with respect to PCR1 reaction's volume, following the manufacturer's instructions. Finally, elute in 50 µL of nuclease-free water.

### PCR2 amplification

53. Prepare the PCR2 reaction on ice as follows (Table 24):

**Table 24**

| **Reaction components** | **Volumes to add** |
|---|---|
| dsDNA | 49 µL |
| Phusion^{™} High-Fidelity PCR Master Mix with HF Buffer* | 50 µL (from the 2X stock) |
| UDI* | 1 µL (from the 10 µM stock) |

| | |
|---|---|
| **Those reagents come from the Phusion*^{™} *High-Fidelity PCR Master Mix with HF Buffer kit - New England Biolabs (M0531S or M0531L).* *^ For the sequences, refer to the tables 26 and 27 "adaptors and primers used for library preparation".* | |

54. Place the tube in a thermal cycler and run the following program (Table 25):

**Table 25**

| **Step** | **Temp** | **Time** |
|---|---|---|
| Initial denaturation | 98 °C | 1 min |
| 5-7 cycles* | 98 °C | 30 sec |
| | 60 °C | 30 sec |
| | 72 °C | 10 sec |
| Hold | 5 °C | ∞ |

| | | |
|---|---|---|
| **The number of cycles depends on the starting material.* | | |

55. Purify the PCR reaction by using NucleoSpin Gel and PCR Clean-Up (MACHEREY-NAGEL, 740609.50) kit following the manufacturer's instructions. Finally, elute in 16 µL of nuclease-free water.

### Optional: Gel-based library purification

56. The dsDNA library could be run on an 8% TAE polyacrylamide gel.

57. Mix the samples with the 6x DNA Loading Dye.

58. Load the samples and the Ultra Low Range DNA Ladder and run the gel at 150 V for 45 minutes.

59. Stain the gel for 5 minutes with SYBR Gold in TAE and visualize the RNA using a UV transilluminator.

60. Excise the library at ~ 200 bp (or ~ 210 bp in the case of the multiplexing protocol) from the gel and dissolve the gel band in 400 µL nuclease-free water, 40 µL ammonium acetate 5 M (in nuclease-free water), and 2 µL SDS 10 % (in nuclease-free water) using a pestle.

61. Leave them overnight at 4 °C in rotation.

62. The following day, transfer the samples into Ultrafree-MC Centrifugal Filter Units and centrifuge them for 3 minutes at 2300 g to separate any gel residue from the DNA.

63. Add 700 µL of isopropanol and 2 µL of Glyco-blue.

64. Put the samples at -80 °C for at least 1 hour.

65. Centrifuge at 10,000-14,000 g for 45 minutes at 4 °C.

66. Wash the pellet with 500 µL of 80% ethanol (in nuclease-free water).

67. Centrifuge at 10,000-14,000 g for 5 minutes at 4 °C.

68. Remove the supernatant and let the pellet air dry for at least 15 minutes.

69. Solubilize the pellets in 15 µL of nuclease-free water.

### Library quality control, quantification, and sequencing

70. Run the samples on the Agilent 2100 Bioanalyzer using High Sensitivity DNA CHIPS (Agilent Technologies #5067-4626) following the manufacturer's instructions.

71. Eventually, analyze the libraries by single-read sequencing 100 cycles with Illumina NovaSeq 6000 (or any other Illumina platform) by the Next Generation Sequencing.

**Table 26**

| ***Ligation*** | | **SEQ ID No.** |
|---|---|---|
| **Adaptor** | NNNNUCUCCUUGCAUAAUCACCAACCAU/idSp//idSp//idSp/ACACGACGCUCUUCCGA UCUNNNN/32FU | 1 |
| **Multiplex ed Adaptor 1** | NNNNUCUCCUUGCAUAAUCACCAACCAU/idSp//idSp//idSp/ACACGACGCUCUUCCGA UCUNNNNGUACCUUG/32FU | 2 |
| **Multiplex ed Adaptor 2** | NNNNUCUCCUUGCAUAAUCACCAACCAU/idSp//idSp//idSp/ACACGACGCUCUUCCGA UCUNNNNUAAUGCCG/32FU | 3 |
| **Multiplex ed Adaptor 3** | NNNNUCUCCUUGCAUAAUCACCAACCAU/idSp//idSp//idSp/ACACGACGCUCUUCCGA UCUNNNNCUCCUAGA/32FU | 4 |
| **Multiplex ed Adaptor 4** | NNNNUCUCCUUGCAUAAUCACCAACCAU/idSp//idSp//idSp/ACACGACGCUCUUCCGA UCUNNNNGAUACUAG/32FU | 5 |
| **Multiplex ed Adaptor 5** | NNNNUCUCCUUGCAUAAUCACCAACCAU/idSp//idSp//idSp/ACACGACGCUCUUCCGA UCUNNNNUAGUACAG/32FU | 6 |
| **Multiplex ed Adaptor 6** | NNNNUCUCCUUGCAUAAUCACCAACCAU/idSp//idSp//idSp/ACACGACGCUCUUCCGA UCUNNNNACGAUACU/32FU | 7 |

| ***Reverse transcription*** | | |
|---|---|---|
| **RT primer** | GTGACTGGAGTTCAGACGTGTGCTCTTCCGATCTNNNNGGTTGGTGATTATGCAAGGAG | 8 |
| **Multiplex ed RT Primer 1** | GTGACTGGAGTTCAGACGTGTGCTCTTCCGATCTNNNNATCATTCCGGTTGGTGATTATGCAA GGAG | 9 |
| **Multiplex ed RT Primer 2** | GTGACTGGAGTTCAGACGTGTGCTCTTCCGATCTNNNNTGGAACAAGGTTGGTGATTATGCAA GGAG | 10 |
| **Multiplex ed RT Primer 3** | GTGACTGGAGTTCAGACGTGTGCTCTTCCGATCTNNNNGTGTTCTAGGTTGGTGATTATGCAA GGAG | 11 |
| **Multiplex ed RT Primer 4** | GTGACTGGAGTTCAGACGTGTGCTCTTCCGATCTNNNNAGTACAAGGGTTGGTGATTATGCAA GGAG | 12 |
| **Multiplex ed RT Primer 5** | GTGACTGGAGTTCAGACGTGTGCTCTTCCGATCTNNNNAGTGGTCAGGTTGGTGATTATGCAA GGAG | 13 |
| **Multiplex ed RT Primer 6** | GTGACTGGAGTTCAGACGTGTGCTCTTCCGATCTNNNNAACGTGATGGTTGGTGATTATGCAA GGAG | 14 |
| **Multiplex ed RT Primer 7** | GTGACTGGAGTTCAGACGTGTGCTCTTCCGATCTNNNNCCGTGAGAGGTTGGTGATTATGCAA GGAG | 15 |
| **Multiplex ed RT Primer 8** | GTGACTGGAGTTCAGACGTGTGCTCTTCCGATCTNNNNGAGTTAGCGGTTGGTGATTATGCAA GGAG | 16 |

| *PCR1* | | |
|---|---|---|
| **Forward PCR1 Primer** | ACACTCTTTCCCTACACGACGCTCTTCCGATCT | 17 |
| **Reverse PCR1 Primer** | GTGACTGGAGTTCAGACGTGT | 18 |

**Table 27**

| ***PCR2*** | | | | |
|---|---|---|---|---|
| | ***i5*** | **SEQ ID No.** | ***i7*** | **SEQ ID No.** |
| UDI Z37 | AATGATACGGCGACCACCGAGATCTACACA GTTACCGAGACACTCTTTCCCTACACGACG CTCTTCCGATCT | 19 | CAAGCAGAAGACGGCATACGAGATCAATGCGACAGT GACTGGAGTTCAGACGTGTGCTCTTCCGATCT | 32 |
| UDI Z38 | AATGATACGGCGACCACCGAGATCTACACG GCTACTTGGACACTCTTTCCCTACACGACG CTCTTCCGATCT | 20 | CAAGCAGAAGACGGCATACGAGATCAACTTGGCAGT GACTGGAGTTCAGACGTGTGCTCTTCCGATCT | 33 |
| UDI Z39 | AATGATACGGCGACCACCGAGATCTACACC CTGTACACCACACTCTTTCCCTACACGACG CTCTTCCGATCT | 21 | CAAGCAGAAGACGGCATACGAGATTCTAGGAGTCGT GACTGGAGTTCAGACGTGTGCTCTTCCGATCT | 34 |
| UDI Z40 | AATGATACGGCGACCACCGAGATCTACACG GTTGACAGGACACTCTTTCCCTACACGACG CTCTTCCGATCT | 22 | CAAGCAGAAGACGGCATACGAGATCCAAGGTTCCGT GACTGGAGTTCAGACGTGTGCTCTTCCGATCT | 35 |
| UDI Z41 | AATGATACGGCGACCACCGAGATCTACACG TCACGTTGTACACTCTTTCCCTACACGACG CTCTTCCGATCT | 23 | CAAGCAGAAGACGGCATACGAGATCTGGTCATCTGT GACTGGAGTTCAGACGTGTGCTCTTCCGATCT | 36 |
| UDI Z42 | AATGATACGGCGACCACCGAGATCTACACC AATGGTCCAACACTCTTTCCCTACACGACG CTCTTCCGATCT | 24 | CAAGCAGAAGACGGCATACGAGATACCATAGGACGT GACTGGAGTTCAGACGTGTGCTCTTCCGATCT | 37 |
| UDI Z1 | AATGATACGGCGACCACCGAGATCTACACA TAAGCGCATACACTCTTTCCCTACACGACG CTCTTCCGATCT | 25 | CAAGCAGAAGACGGCATACGAGATCTTCGCAACTGT GACTGGAGTTCAGACGTGTGCTCTTCCGATCT | 38 |
| UDI Z2 | AATGATACGGCGACCACCGAGATCTACACT GGTGAAGTGACACTCTTTCCCTACACGACG CTCTTCCGATCT | 26 | CAAGCAGAAGACGGCATACGAGATCGTCAAGACGGT GACTGGAGTTCAGACGTGTGCTCTTCCGATCT | 39 |
| UDI Z5 | AATGATACGGCGACCACCGAGATCTACACT TACACGGTTACACTCTTTCCCTACACGACG CTCTTCCGATCT | 27 | CAAGCAGAAGACGGCATACGAGATCCACATTGCCGT GACTGGAGTTCAGACGTGTGCTCTTCCGATCT | 40 |
| UDI Z6 | AATGATACGGCGACCACCGAGATCTACACA GCAGTGAAGACACTCTTTCCCTACACGACG CTCTTCCGATCT | 28 | CAAGCAGAAGACGGCATACGAGATACGTCCAAACGT GACTGGAGTTCAGACGTGTGCTCTTCCGATCT | 41 |
| UDI Z46 | AATGATACGGCGACCACCGAGATCTACACC TCGTGTACTACACTCTTTCCCTACACGACG CTCTTCCGATCT | 29 | CAAGCAGAAGACGGCATACGAGATTCACTCGATCGT GACTGGAGTTCAGACGTGTGCTCTTCCGATCT | 42 |
| UDI Z3 | AATGATACGGCGACCACCGAGATCTACACC CCCTATACCACACTCTTTCCCTACACGACG CTCTTCCGATCT | 30 | CAAGCAGAAGACGGCATACGAGATTGTCACACTGGT GACTGGAGTTCAGACGTGTGCTCTTCCGATCT | 43 |
| UDI Z15 | AATGATACGGCGACCACCGAGATCTACACT GGTACCTTGACACTCTTTCCCTACACGACG CTCTTCCGATCT | 31 | CAAGCAGAAGACGGCATACGAGATATCCTTCCATGT GACTGGAGTTCAGACGTGTGCTCTTCCGATCT | 44 |

### REFERENCES

- Clamer, Massimiliano, et al. "Active ribosome profiling with RiboLace." Cell Reports 25.4 (2018): 1097-1108.
- Froberg, John E., Omer Durak, and Jeffrey D. Macklis. "Development of nanoRibo-seq enables study of regulated translation by cortical neuron subtypes, showing uORF translation in synaptic-axonal genes." Cell reports 42.9 (2023).
- Heiman, Myriam, et al. "A translational profiling approach for the molecular characterization of CNS cell types." Cell 135.4 (2008): 738-748.
- Hornstein, Nicholas, et al. "Ligation-free ribosome profiling of cell type-specific translation in the brain." Genome biology 17 (2016): 1-15.
- Ingolia, Nicholas T., et al. "Genome-wide analysis in vivo of translation with nucleotide resolution using ribosome profiling." Science 324.5924 (2009): 218-223.
- Ingolia, Nicholas T. "Ribosome footprint profiling of translation throughout the genome." Cell 165.1 (2016): 22-33.
- Khitun, Alexandra, Travis J. Ness, and Sarah A. Slavoff. "Small open reading frames and cellular stress responses." Molecular omics 15.2 (2019): 108-116.
- Lauria, Fabio, et al. "riboWaltz: optimization of ribosome P-site positioning in ribosome profiling data." PLoS computational biology 14.8 (2018): e1006169
- Lauria, Fabio, et al. "SMN-primed ribosomes modulate the translation of transcripts related to spinal muscular atrophy." Nature cell biology 22.10 (2020): 1239-1251.
- Li, Qianru, et al. "Low-input RNase footprinting for simultaneous quantification of cytosolic and mitochondrial translation." Genome research 32.3 (2022): 545-557.
- Liu, Qi, et al. "Ribo-uORF: a comprehensive data resource of upstream open reading frames (uORFs) based on ribosome profiling." Nucleic Acids Research 51.D1 (2023): D248-D261.
- McGlincy, Nicholas J., and Nicholas T. Ingolia. "Transcriptome-wide measurement of translation by ribosome profiling." Methods 126 (2017): 112-129.
- Mehta, Preeti, et al. "Ribosome purification approaches for studying interactions of regulatory proteins and RNAs with the ribosome." Bacterial Regulatory RNA: Methods and Protocols (2012): 273-289.
- Meindl, Andreas, et al. "A rapid protocol for ribosome profiling of low input samples." Nucleic acids research 51.13 (2023): e68-e68.
- Migazzi, Alice, et al. "Huntingtin-mediated axonal transport requires arginine methylation by PRMT6." Cell reports 35.2 (2021).
- Moro, Simone G., et al. "Impact of uORFs in mediating regulation of translation in stress conditions." BMC molecular and cell biology 22 (2021): 1-13.
- Mudge, Jonathan M., et al. "Standardized annotation of translated open reading frames." Nature biotechnology 40.7 (2022): 994-999.
- Ozadam H, Tonn T, Han CM, Segura A, Hoskins I, Rao S, Ghatpande V, Tran D, Catoe D, Salit M, Cenik C. Single-cell quantification of ribosome occupancy in early mouse development. Nature. 2023 Jun;618(7967):1057-1064.
- Rivera, Maria C., Bruce Maguire, and James A. Lake. "Isolation of ribosomes and polysomes." Cold Spring Harbor Protocols 2015.3 (2015): pdb-prot081331.
- Silva, Joana, Rafael Fernandes, and Luísa Romão. "Translational regulation by upstream open reading frames and human diseases." The mRNA Metabolism in Human Disease (2019): 99-116.
- Tebaldi, Toma, et al. "Widespread uncoupling between transcriptome and translatome variations after a stimulus in mammalian cells." BMC genomics 13 (2012): 1-15.
- VanInsberghe, Michael, et al. "Single-cell Ribo-seq reveals cell cycle-dependent translational pausing." Nature 597.7877 (2021): 561-565.
- Vatikioti, Alexandra, et al. "Translational regulation and deregulation in erythropoiesis." Experimental Hematology 75 (2019): 11-20.
- Wang Q and Mao Y. "Principles, challenges, and advances in ribosome profiling: from bulk to low-input and single-cell analysis." Advanced Biotechnology 1.4 (2023): 6.
- Xiong, Zhuging, et al. "Ultrasensitive Ribo-seq reveals translational landscapes during mammalian oocyte-to-embryo transition and pre-implantation development." Nature Cell Biology 24.6 (2022): 968-980.
- Zhang C, Wang M, Li Y, Zhang Y. Profiling and functional characterization of maternal mRNA translation during mouse maternal-to-zygotic transition. Sci Adv. 2022 Feb 4;8(5):eabj3967. doi: 10.1126/sciadv.abj3967.

## Claims

1. Method for ligating an adaptor to a ribosome-embedded RNA Fragment (REF) from a cell or tissue population, the method comprising the following steps:
(i) performing a lysis of the cell or tissue population, obtaining a lysate;
(ii) digesting the lysate with an endoribonuclease, obtaining a digested lysate;
(iii) inhibiting the endoribonuclease in the digested lysate, obtaining an inhibited lysate;
(iv) incubating the inhibited lysate with an adaptor in conditions to allow ligation of the adaptor to the ribosome-embedded RNA fragment (REF), and obtaining an adaptor-REF complex, wherein the adaptor has formula (I):
5' OH-Nₓ-C1-L1-A_{z}-PR1-N_{y}-C2-B-OH 3' (I)
wherein
- N is a ribonucleotide,
- x and y are integer numbers independently selected from 1 to 20,
- L1 is an oligoribonucleotide sequence having a length comprised between 15 and 30 nucleotides,
- A is an abasic site or a spacer allowing the arrest of a retrotranscriptase enzyme activity,
- z is an integer number from 1 to 5,
- PR1 is a first portion of a first nucleic acid domain of a sequencing platform adapter construct, having a length comprised between 10 and 80 nucleotides,
- B is none, or a ribonucleotide having a 2'-fluoro-base, or a ribonucleotide having a modified nucleobase conferring nuclease resistance, and
- C1 and C2 are none or a barcode sequence comprising up to 20 nucleotides, provided that at least one between C1 and C2 is none.

2. The method according to claim 1, further comprising a purification step (v) of the adaptor-REFs complex.

3. The method according to claim 1 or claim 2, wherein the PR1 nucleic acid domain is derived from the N-term portion of the sequences selected from: *(i)* P1 adaptor, *(ii)* GS adaptor A, *(iii)* MGI 5' adapter, and *(iv)* SP1 sequences.

4. The method according to any one of the preceding claims, wherein, before the lysis step (i), the cell population is treated with an inhibitor of translation elongation to arrest ribosomes on mRNAs at their precise positions at the time of treatment (step (0a)) or with an inhibitor of translation initiation to arrest ribosomes only at translation start sites (step (0b)).

5. The method according to any one of the preceding claims, wherein, when the method is carried out in well plates, the steps (ii) and (iii) can be combined into a single step.

6. The method according to any one of claims 1 to 4, wherein, before the incubation step (iv), a ribosome isolation step (iiia) is carried out.

7. The method according to any one of the preceding claims, wherein, when a further step of ribosome isolation (step (iiia)) is carried out, the ligation step (iv) is carried out using a ligation buffer comprising: a buffer solution, a divalent chloride salt, a monovalent chloride salt, a reducing agent, an inhibitor of translation elongation, an RNase inhibitor, a ribonucleotide triphosphate, a divalent cation cofactor, an RNA ligation enzyme, and the adaptor of formula (I).

8. The method according to any one of claims 1 to 6, wherein, when a further step of ribosome isolation (step (iiia)) is not carried out, the ligation step (iv) is carried out using a ligation buffer comprising: a buffer solution, a divalent chloride salt, a monovalent chloride salt, a reducing agent, an inhibitor of translation elongation, an RNase inhibitor, a ribonucleotide triphosphate, a divalent cation cofactor, an RNA ligation enzyme, a non-ionic detergent, a deoxyribonuclease and the adaptor of formula (I).

9. The method according to any one of the preceding claims, wherein, when more than one cell population is employed, the adaptor-REF complexes of each cell population obtained at the end of the ligation step (iv) are pooled together, wherein the domains C1 or C2 of the adaptor of formula (I) have a nucleotide sequence different for each cell population.

10. A kit suitable for implementing the method according to any one of the preceding claims for ligating an adaptor to a REF, the kit comprising:
(a) an adaptor of formula (I):
5' OH-Nₓ-C1-L1-A_{z}-PR1-N_{y}-C2-B-OH 3' (I)
wherein
- N is a ribonucleotide,
- x and y are integer numbers independently selected from 1 to 20,
- L1 is an oligoribonucleotide sequence having a length comprised between 15 and 30 nucleotides,
- A is an abasic site or a spacer allowing the arrest of a retrotranscriptase enzyme activity,
- z is an integer number from 1 to 5,
- PR1 is a first portion of a first nucleic acid domain of a sequencing platform adapter construct, having a length comprised between 10 and 80 nucleotides,
- B is none, or a ribonucleotide having a 2'-fluoro-base, or a ribonucleotide having a modified nucleobase conferring nuclease resistance, and
- C1 and C2 are none or a barcode sequence comprising up to 20 nucleotides, provided that at least one between C1 and C2 is none;
(b) an endonuclease enzyme;
(c) an inhibitor of the endonuclease enzyme; and
(d) a ligase enzyme.

11. The kit according to claim 10, wherein the PR1 nucleic acid domain is derived from the N-term portion of the sequences selected from: *(i)* P1 adaptor, *(ii)* GS adaptor A, *(iii)* MGI 5' adapter, and *(iv)* SP1 sequences.

12. The kit according to claim 10 or claim 11, wherein the endonuclease enzyme is an endoribonuclease enzyme suitable to hydrolyze single-stranded RNA into nucleoside 3'-monophosphate or cyclic phosphate.
